Europäisches Patentamt

European Patent Office

Office européen des brevets

(19)

(11) Publication number: **0 212 531**
A1

(12) # EUROPEAN PATENT APPLICATION

(21) Application number: **86111097.1**

(22) Date of filing: **11.08.86**

(51) Int. Cl.⁴: **C 07 K 7/10, C 12 P 21/02, C 12 N 15/00, A 61 K 37/43**

(30) Priority: **12.08.85 US 765037**

(43) Date of publication of application: **04.03.87 Bulletin 87/10**

(84) Designated Contracting States: **AT BE CH DE FR GB IT LI LU NL SE**

(71) Applicant: **SYNTEX (U.S.A.) INC., 3401 Hillview Avenue, Palo Alto, California 94304 (US)**

(72) Inventor: **Baecker, Preston, 1644 Grosbeak, Sunnyvale California 94087 (US)**
Inventor: **Bach, Chinh, 750 Creekland Circle, San Jose California 95133 (US)**
Inventor: **Chan, Hardy, 2755 Saint James Road, Belmont California 94022 (US)**

(74) Representative: **Barz, Peter, Dr. et al, Patentanwälte Dipl.-Ing. G. Dannenberg Dr. P. Weinhold, Dr. D. Gudel Dipl.-Ing. S. Schubert, Dr. P. Barz Siegfriedstrasse 8, D-8000 München 40 (DE)**

(54) **Bovine growth hormone releasing factor derivative.**

(57) Administration of BGRF$_{Leu27}$ stimulates release of Growth Hormone, which increases weight gain in animals and milk production in lactating mammals.

EP 0 212 531 A1

-1-

## BOVINE GROWTH HORMONE RELEASING FACTOR DERIVATIVE

This invention relates to a new derivative of bovine growth hormone releasing factor (BGRF) in which Met27 is replaced with Leu, namely ($BGRF_{Leu27}$). This invention also relates to a method of increasing weight gain in animals and increasing milk production in lactating mammals.

Growth hormone releasing factors (GRFs) are known to stimulate the release of growth hormone in animals.

Certain growth hormone releasing factors and derivatives are known. See, for example, U.S. Pat. Nos. 4,529,595, 4,528,190, 4,518,586, and EPO Applications Nos. 137,689 and 138,416.

$BGRF_{Leu27}$ lacks the position 27 methionine, thus allowing the use of cyanogen bromide digestion to cleave a BGRF fusion protein without loss of activity. Additionally, $BGRF_{Leu27}$ is more stable to oxidation than the GRF compounds of the art. It has also surprisingly been found that $BGRF_{Leu27}$ is active across species, i.e., $BGRF_{Leu27}$ may be administered to non-bovine animals and will induce the release of growth hormone as if the native GRF had been administered.

The following terms appearing in the Specification and appended Claims are defined as follows:

The term "GRF" stands for growth hormone releasing factor. HuGRF denotes human growth hormone releasing factor, whereas PGRF and BGRF denote the porcine and bovine varieties, respectively.

The term "effective amount" of $BGRF_{Leu27}$ refers to the amount of $BGRF_{Leu27}$ that must be administered to an appropriate subject in order to effect a measurable increase in weight gain or milk yield.

The term "sequence," as applied to a DNA sequence or a polypeptide sequence, refers to a DNA molecule or a polypeptide molecule in which the nucleotide or amino acid monomers are arranged in a particular order.

A term in the form "TrpLE·(restriction enzyme)" refers to the TrpLE deletion mutant DNA sequence from the 5' end up to the corresponding restriction enzyme recognition site. Thus, the term "TrpLE·Sst II" refers to the E. coli DNA sequence encoding the Trp promoter and the LE protein up to the first Sst II restriction site. Similarly, the term "TrpLE·BssH II" refers to the E. coli DNA sequence encoding the TrpLE protein up to the first BssH II restriction site.

A term in the form "TrpLE·(restriction enzyme)-(heterologous sequence)" refers to the TrpLE deletion mutant DNA sequence encoding, from 5' to 3', the Trp promoter and the TrpLE protein up to the first corresponding restriction enzyme recognition site named, optionally followed by a cleavable linker sequence, with the heterologous DNA coding for the desired heterologous protein inserted at the 3' end of the of the cleavable linker sequence (if present), or at the 3' end of the TrpLE sequence. For example, the term "TrpLE·Sst II-HuGRF" refers to the DNA sequence which codes for the

0212531

truncated LE protein, optionally a cleavable linker sequence, and human growth hormone releasing factor.

A term in the form "LE-(heterologous polypeptide)" refers to the fusion protein produced by expressing the DNA sequence TrpLE·(restriction enzyme)-(heterologous sequence). For example, LE-HuGRF$_{Leu27}$ refers to the fusion protein obtained by expressing either TrpLE·Sst II-HuGRF$_{Leu27}$ (comprising the LE polypeptide up to the Sst II site), or TrpLE·BssH II-HuGRF$_{Leu27}$ (comprising the LE polypeptide up to the BssH II site), optionally an oligopeptide encoded by a cleavable linker, and human growth releasing factor modified by replacing the amino acid at position 27 with leucine.

The term "cleavable linker sequence" refers to a nucleotide sequence which encodes for a peptide sequence which is easily cleavable by methods known in the art. For example, polypeptides containing methionine are easily cleaved using cyanogen bromide (CNBr), Asp-Pro peptide sequences are cleaved by acid hydrolysis, and Asp-Asp-Asp-Asp-Lys sequences are cleaved by the endopeptidase enterokinase. See, e.g., A. Light, et al., Anal. Biochem., 106, 199-206 (1980). To avoid denaturing the heterologous polypeptide, one should avoid using cleavable linker sequences which encode cleavable peptide sequences that are also present in the heterologous polypeptide. For example, if the heterologous protein contains internal Asp-Pro links, it is preferred to use a cleavable linker sequence that does not code for Asp-Pro.

The term "heterologous sequence" refers to a DNA molecule which encodes a protein or protein segment not native to the expression vector. Exemplary heterologous sequences encode human, bovine, or porcine growth hormone releasing factors.

As applied to a cleaved dsDNA segment, the term "3' end" refers to the end of the dsDNA segment which would correspond to the 3' end of the mRNA which would be transcribed from the "sense" or coding strand.

One aspect of the invention is the polypeptide bovine growth hormone releasing factor in which Met27 is replaced with Leu ($BGRF_{Leu27}$). In this compound, the carboxy end group may be in the free acid (-OH) or amide ($-NH_2$) form.

Another aspect of the invention is a method for producing $BGRF_{Leu27}$, which method comprises selecting a plasmid containing a suitable native DNA sequence, cleaving the native sequence with a restriction endonuclease, inserting a $BGRF_{Leu27}$ DNA sequence within the native sequence in the correct reading frame to produce a plasmid encoding a native polypeptide fragment-$BGRF_{Leu27}$ fusion protein, transforming a suitable bacterial host with the resulting fusion protein-encoding plasmid, expressing the native polypeptide fragment-$BGRF_{Leu27}$ fusion protein, and cleaving the fusion protein to release $BGRF_{Leu27}$.

Another aspect of the invention is a plasmid expression vector for producing $BGRF_{Leu27}$, which vector comprises a plasmid containing a suitable native DNA sequence and a $BGRF_{Leu27}$ DNA sequence in the same reading frame and continuous with said native DNA sequence.

Another aspect of the invention is a method for increasing weight gain in animals, which method comprises administering to an animal an effective amount of $BGRF_{Leu27}$.

Another aspect of the invention is a method for increasing milk production in lactating mammals, which method comprises administering to a mammal an effective amount of BGRF$_{Leu27}$.

The manner in which these and other objects and advantages of the invention are obtained will become more apparent from the detailed description which follows and from the accompanying drawings, in which:

Figure 1 illustrates the construction of an intermediate vector plasmid pTrpE-#22.

Figure 2 illustrates the construction of the expression vector pTrpLE-#14.

Figure 3 illustrates the DNA and amino acid sequences predicted to encode the mRNA produced from the plasmid pTrpLE·Sst 11-HuGRF$_{Leu27}$-A and plasmid pTrpLE·Sst II-HuGRF$_{Leu27}$-B. The terminal sequences are based on information available in the literature. Amino acids of the HuGRF segment are shown in capital letters.

Figure 4 illustrates a flow chart of the purification scheme used to produce HuGRF$_{Leu27}$ from E. coli cells.

Figure 5 depicts an SDS protein gel demonstrating high production of the TrpLE, TrpLE-BssH II-HuGRF$_{Leu27}$ and TrpLE-Sst II-HuGRF$_{Leu27}$ proteins from E. coli cells.

Figure 6 depicts an SDS protein gel demonstrating the purification of TrpLE-BssH II-HuGRF$_{Leu27}$ from E. coli by cell lysis and centrifugation before CNBr cleavage.

Figure 7 depicts HPLC profiles of peptides released by CNBr treatment of TrpLE-Sst II-HuGRF$_{Leu27}$ (A) and TrpLE-BssH II-HuGRF$_{Leu27}$ (B). The HPLC fractions in (B) were assayed for GRF activity by activation of adenylate cyclase, and the results presented in (C). The arrow in (A) indicates the fraction with peak activity.

Figure 8 depicts a western blot of TrpLE-BssH II-HuGRF$_{Leu27}$ (A) and TrpLE-Sst II-HuGRF$_{Leu27}$ (B) using anti-HuGRF antisera, after CNBr cleavage and HPLC fractionation of the fusion proteins.

Figure 9 illustrates the purity of several HuGRF$_{Leu27}$ preparations by Coomassie Brilliant Blue stain (A), and western blotting with anti-HuGRF antisera. Authentic HuGRF (S) is compared to HuGRF$_{Leu27}$ purified from two lots of TrpLE-BssH II-HuGRF$_{Leu27}$ (lanes 1, 2) and TrpLE-Sst II-HuGRF$_{Leu27}$ (lane 3). Unmarked lanes contain molecular weight standards.

Figure 10 depicts the HPLC profiles of lots 1, 2, and 3 described in Figure 9.

Figure 11 depicts the HPLC profile of BGRF$_{Leu27}$.

One aspect of the invention is the polypeptide bovine growth hormone releasing factor in which Met27 is replaced with Leu (BGRF$_{Leu27}$).

Another aspect of the invention is a method for producing BGRF$_{Leu27}$, which method comprises:

selecting a plasmid containing a suitable native DNA sequence;

cleaving the native sequence with a restriction endonuclease;

inserting a BGRF$_{Leu27}$ DNA sequence within the native sequence in the correct reading frame to produce a plasmid encoding a native polypeptide fragment-BGRF$_{Leu27}$ fusion protein;

transforming a suitable bacterial host with the resulting fusion protein-encoding plasmid;

expressing the native polypeptide fragment-BGRF$_{Leu27}$ fusion protein; and

87131                                                    25190-FF

cleaving the fusion protein to release $BGRF_{Leu27}$.

A preferred subgenus is the method wherein the bacterial host is E. coli. A preferred class is the method which further comprises inserting a cleavable linker DNA sequence between said native DNA sequence and said $BGRF_{Leu27}$ DNA sequence. A preferred subclass is the method wherein said cleavable linker encodes Met, Asp-Pro, or Asp-Asp-Asp-Asp-Lys. A preferred species is the method wherein the suitable native DNA sequence is TrpLE.

Another aspect of the invention is a plasmid expression vector for producing $BGRF_{Leu27}$, which vector comprises a plasmid containing a suitable native DNA sequence and a $BGRF_{Leu27}$ DNA sequence in the same reading frame and continuous with said native DNA sequence. A preferred subgenus is the vector which further comprises a cleavable linker DNA sequence between said native DNA sequence and said $BGRF_{Leu27}$ DNA sequence. A preferred class is the vector wherein said cleavable linker encodes Met, Asp-Pro, or Asp-Asp-Asp-Asp-Lys. A preferred subclass is the vector wherein said native DNA sequence is TrpLE.

Another aspect of the invention is a method for increasing weight gain in animals, which method comprises administering to an animal an effective amount of $BGRF_{Leu27}$. A preferred species is the method wherein said animal is bovine. Another preferred species is the method wherein said animal is porcine. Another preferred species is the method wherein said animal is poultry.

Another aspect of the invention is a method for increasing milk production in lactating mammals, which method comprises administering to a mammal an effective amount of $BGRF_{Leu27}$. A preferred species is the method wherein said mammal is bovine.

## TrpLE

The fusion between the DNA sequences encoding the LE protein and the heterologous polypeptide is made in a manner such that various distal portions of the LE protein will be replaced, in the same translational reading frame, by the heterologous polypeptide. Additionally, other amino acid insertions, deletions and substitutions in the LE protein may be introduced so as to facilitate the purification of the desired product.

Cells are transformed by addition of the expression vehicles of the invention and grown under conditions in which the exogenous promoter-operator is repressed. For instance, in the case of the E. coli tryptophan promoter-operator (Trp promoter), cells are grown in the presence of added tryptophan. This represses the Trp promoter such that cell growth can proceed normally, without deleterious effect due to overexpression of the fusion protein. When the recombinant culture reaches a cell density appropriate for industrial production of the polypeptide, the external source of repression (e.g., tryptophan) is removed, or, alternatively, an external inducer (e.g., indole acrylic acid) is added. This derepresses the exogenous promoter-operator (e.g., Trp promoter) and highly efficient expression of the heterologous insert occurs.

The invention provides a variety of useful intermediates and end products, including heterologous polypeptide-containing fusion proteins which can be easily separated from the remainder of the bacterial proteins by virtue of the self-aggregating properties conferred by the LE protein portion. The fusion protein may then be specifically cleaved to release the desired heterologous polypeptide, whose purification is facilitated by the choice of the fusion site within the LE protein. Alternatively, one may use the fusion protein without cleavage, e.g., to produce vaccines or monoclonal antibodies for immunoassays.

The original isolation of the TrpLE protein cloning region was the result of genetic manipulations of the E. coli tryptophan operon. See K. Bertrand, C. Squires and C. Yanofsky, J. Mol. Biol., 103, 319-337 (1976); G. F. Miozzari and C. Yanofsky, J. Bact., 133, 1457-1466 (1978). One of these original deletions, TrpLE1413, is available on plasmid pVV1 (B. P. Nichols and C. Yanofsky, Meth. Enzymol., 101, 155-164 (1983)) and was used to construct the LE protein vectors employed in the examples herein. The essential features of a DNA deletion required to form an effective LE protein vector for the purposes of this invention are, relative to the Trp operon (C. Yanofsky et al., Nuc. Acids Res., 9, 6647-6668 (1981)):

a 5' terminal breakpoint near a base coding for amino acid #10 of the TrpL peptide;

a 3' terminal breakpoint about two-thirds of the way through the TrpE coding region, preferably near amino acid #339.

Such a deletion removes the Trp operon attenuator region, allowing for higher level of gene expression, and preserves a region of the TrpE protein from about amino acid #339 to the carboxy terminal amino acid, which is preferred for its ability to self-aggregate even when heterologous proteins are substituted for a portion of the carboxy coding region.

A preferred embodiment of the invention is the LE vector plasmid pTrpLE-#14. This is a pBR322-based plasmid containing the E. coli Trp operon and LE coding region of 191 amino acids from pVV1, followed in turn by a portion of the TrpD gene and a transcription terminator fragment from the E. coli RNA polymerase-β subunit gene (RpoC: See C. Squires et al., Nucleic Acids Res., 9, 6827-6840 (1981)). The terminator provides for termination of mRNA synthesis, thereby preventing transcription beyond the gene of interest which could interfere with plasmid replication or stability. Transcripts of the terminator region may also form structures which stabilize the 3' end of the mRNA against degradation.

The expression of the LE protein either alone or fused to a heterologous gene is conveniently brought about by the Tryptophan promoter in plasmid pTrpLE-#14 and its derivatives as described herein. It is appreciated, however, that any similar well-characterized promoter-operator DNA region known to be capable of regulating gene expression in E. coli could be substituted for the Trp operon promoter used in these particular examples. The promoter region of the Trp operon commends itself for this use because it is well-characterized (I. P. Crawford and G. V. Stauffer, Ann. Rev. Biochem., 49, 163-195 (1980)) and can be easily induced to produce high levels of mRNA and hence protein products by growth in media either lacking tryptophan or containing the inducer compound indole acrylic acid (IAA). Other suitable control elements might include the following, or selected regions thereof; the Lac promoter, the Tac promoter, the Arabinose, Galactose or Alkaline Phosphatase operons, or a promoter-operator from bacteriophage lambda.

Likewise, the provision of a transcription termination function by the RpoC DNA fragment and the selectable marker of ampicillin resistance employed in pTrpLE-#14 were used by way of an example and are not meant to limit the scope of this invention.

The choice of the preferred expression vectors discussed above gives good yields of fusion product, transcribed/translated with high efficiency. The protein

87131                                        25190-FF

product is insoluble, is easy to process, and provides protection from proteolysis in the cell.

GRF

Growth hormone releasing factor (GRF) is a small (44 amino acid) polypeptide secreted by the pituitary which stimulates the release of growth hormone in mammals. GRFs from different species typically vary by a few amino acids in composition, as illustrated below:

|       | 1   |     |     |     | 5   |     |     |     |     | 10  |
|-------|-----|-----|-----|-----|-----|-----|-----|-----|-----|-----|
| HuGRF: | Tyr | Ala | Asp | Ala | Ile | Phe | Thr | Asn | Ser | Tyr |
| PGRF : | Tyr | Ala | Asp | Ala | Ile | Phe | Thr | Asn | Ser | Tyr |
| BGRF : | Tyr | Ala | Asp | Ala | Ile | Phe | Thr | Asn | Ser | Tyr |

|     |     |     |     | 15  |     |     |     |     | 20  |
|-----|-----|-----|-----|-----|-----|-----|-----|-----|-----|
| Arg | Lys | Val | Leu | Gly | Gln | Leu | Ser | Ala | Arg |
| Arg | Lys | Val | Leu | Gly | Gln | Leu | Ser | Ala | Arg |
| Arg | Lys | Val | Leu | Gly | Gln | Leu | Ser | Ala | Arg |

|     |     |     |     | 25  |     | 27  |     |     | 30  |
|-----|-----|-----|-----|-----|-----|-----|-----|-----|-----|
| Lys | Leu | Leu | Gln | Asp | Ile | Met | Ser | Arg | Gln |
| Lys | Leu | Leu | Gln | Asp | Ile | Met | Ser | Arg | Gln |
| Lys | Leu | Leu | Gln | Asp | Ile | Met | Asn | Arg | Gln |

|     |     |     |     | 35  |     |     |     |     | 40  |
|-----|-----|-----|-----|-----|-----|-----|-----|-----|-----|
| Gln | Gly | Glu | Ser | Asn | Gln | Glu | Arg | Gly | Ala |
| Gln | Gly | Glu | Arg | Asn | Gln | Glu | Gln | Gly | Ala |
| Gln | Gly | Glu | Arg | Asn | Gln | Glu | Gln | Gly | Ala |

|     |     |     | 44  |        |
|-----|-----|-----|-----|--------|
| Arg | Ala | Arg | Leu | $NH_2$ |
| Arg | Val | Arg | Leu | $NH_2$ |
| Lys | Val | Arg | Leu | $NH_2$ |

Production in E. coli of human growth hormone releasing factor (HuGRF) was achieved by fusing the HuGRF DNA sequence to the TrpLE sequence. In a preferred embodiment, the fusion between the HuGRF and the LE protein coding regions was via the codon for methionine (ATG), to allow for release of the HuGRF via specific cleavage at Met using CNBr. To prevent cleavage at the methionine in position 27 of HuGRF, position 27 was changed to a leucine in the synthetic DNA which was cloned into the LE protein vector. Calculations predicted that substitution of Leu for Met would result in the least change in secondary structure and hydrophobicity (P.Y. Chou & G.D. Fasman, Adv. in Enzymol., 47, 45-148 (1978)). However, other naturally occuring amino acids may be substituted to prevent CNBr cleavage. The synthetic DNA was also designed to maximize the use of amino acid codons which are favored in highly expressed E. coli proteins (R. Grantham et al., Nucleic Acids Res., 9, r43-r74 (1981)), to minimize potential secondary structure in the resultant mRNA, and to provide convenient restriction sites which are useful for screening the desired clone and facilitating future alternatives in the DNA sequence. The synthetic DNA coding for the HuGRF$_{Leu27}$ was ligated to either the Sac II site of the LE gene, replacing the carboxy terminal 73 amino acids of the LE protein, or to the BssH II site, replacing 11 amino acids.

Although the examples cited interpose a methionine between the LE protein and the heterologous polypeptide, it will be appreciated that this junction may comprise any combination of the twenty naturally occuring amino acids, or none at all, and need not be specifically cleavable by chemical or enzymatic means, yet still remain within the scope of the invention.

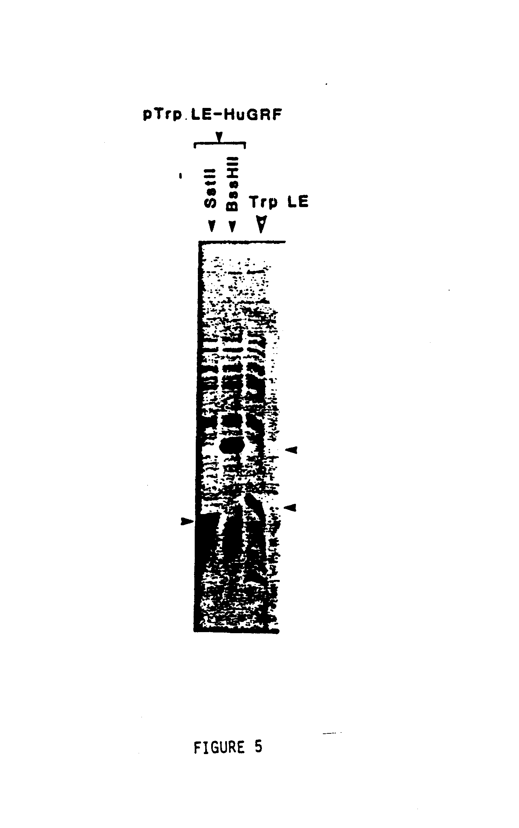

Good induction of expression from the Trp operon of plasmids encoding the HuGRF$_{Leu27}$ fused to either the BssH II or Sac II sites of the LE gene was readily achieved (Figure 5). The kinetics of synthesis and the extent of accumulation of the HuGRF$_{Leu27}$ were dependent upon the composition of the culturing media. Indole acrylic acid (IAA) was required to maximally induce synthesis in a relatively rich medium, L broth. In contrast, inclusion of IAA in a minimal medium, M-9, altered only the kinetics of accumulation, but not the

87131                                                        25190-FF

final level, of the LE-HuGRF$_{Leu27}$. As much as 35% of total protein was LE-HuGRF$_{Leu27}$ after growth in M-9, compared to 15-20% in L broth plus IAA. Maximal stability of the plasmids is obtained by growth in media containing 20-100 µg/ml tryptophan until expression is desired.

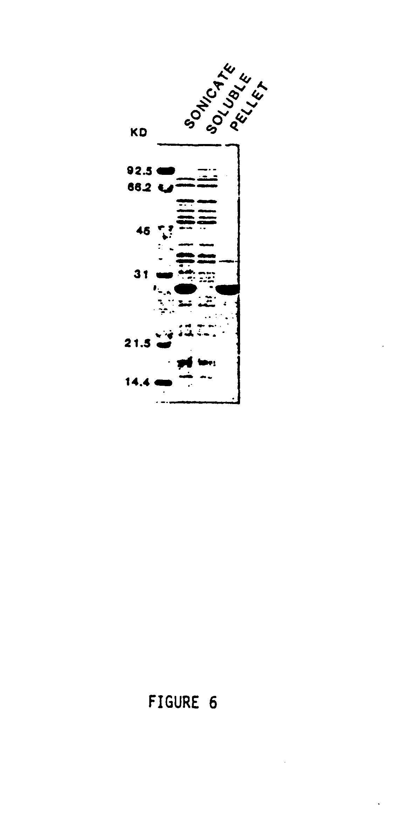

Purification of the HuGRF$_{Leu27}$ was commenced by recovery of the insoluble aggregate of LE-HuGRF$_{Leu27}$ protein from the lysed cells by low speed centrifugation (Figure 6). This material was treated with CNBr in 70% formic acid. Formic acid serves as a preferred solvent to dissolve the LE fusion protein aggregates in the pellet and thereby provide a homogenous solution for further processing. Where the fusion protein itself is the desired product, solvents such as SDS or urea may be used to dissolve the LE fusion protein aggregates prior to further processing. After removal of excess CNBr and formic acid, the HuGRF$_{Leu27}$ peptide was extracted from the insoluble material with 1N acetic acid. The extract was then dialyzed into dilute buffer before fractionation by reverse phase HPLC.

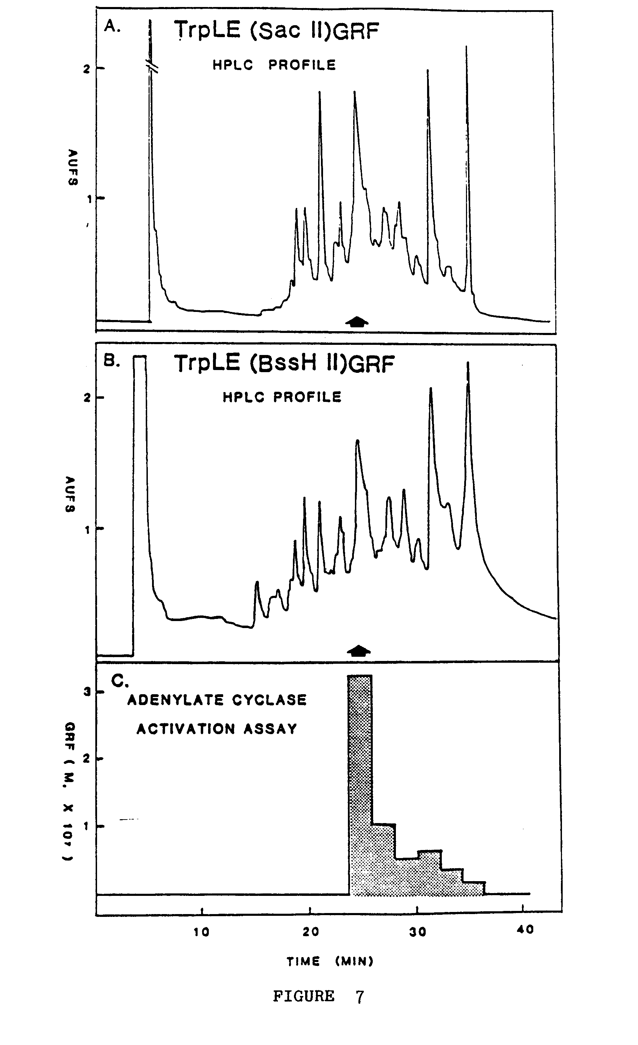

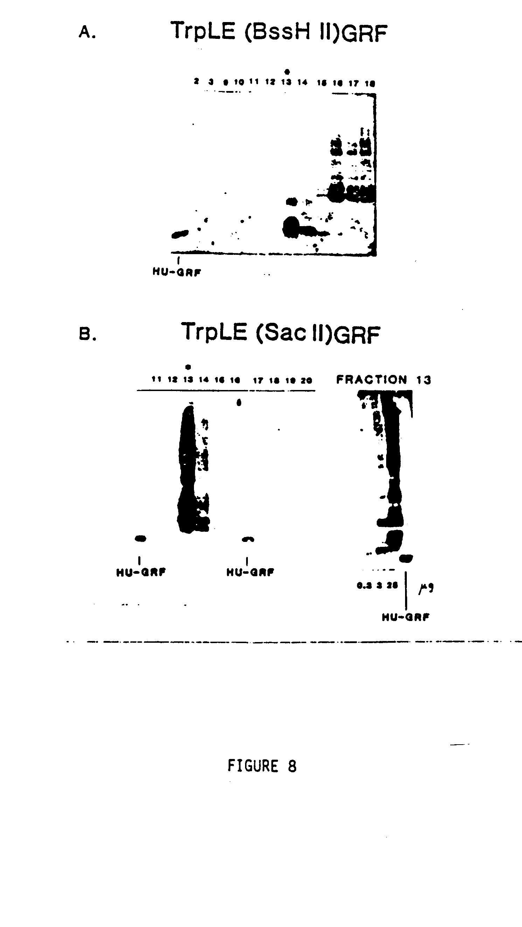

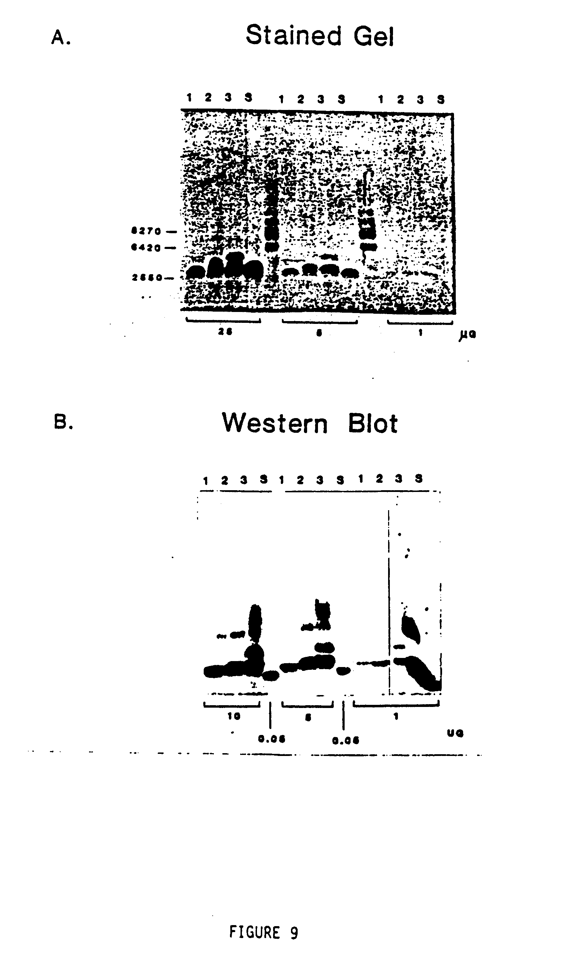

Good resolution of HuGRF$_{Leu27}$ has been achieved using either reverse phase HPLC or a combination of CM-cellulose and HPLC (Figures 7 and 8). The latter approach was preferred for material isolated from the TrpLE·Sac II-HuGRF$_{Leu27}$ clone since CM-cellulose removed some contaminants which were not possible to separate by HPLC alone. Fractionation of the dialysate prepared from the TrpLE·BssH II-HuGRF$_{Leu27}$ clone (Fig. 7B) or the TrpLE·Sac II-HuGRF$_{Leu27}$ clone (Fig. 7A) on HPLC gave HPLC profiles of total protein that were nearly identical. In contrast, when analyzed by the sensitive western blotting technique (Fig. 8B), the HuGRF$_{Leu27}$ from the Sac II clone was shown to elute from the HPLC with immunoreactive material of higher molecular weight. When the dialysate from the Sac II cloned HuGRF$_{Leu27}$ was first fractionated on CM-cellulose, the HPLC-purified material was substantially free of these contaminants (Figure 9B). HuGRF$_{Leu27}$ of the greatest purity was isolated from the BssH II clone, even without a CM-cellulose step, because the immunoreactive contaminants were well separated by HPLC (Fig. 8A). The identity of these immunoreactive products was based upon their co-migration with the immunoreactive products seen with limiting amounts of CNBr, their ability to stimulate adenylate cyclase in an in vitro GRF assay, and their reactivity with anti-HuGRF sera.

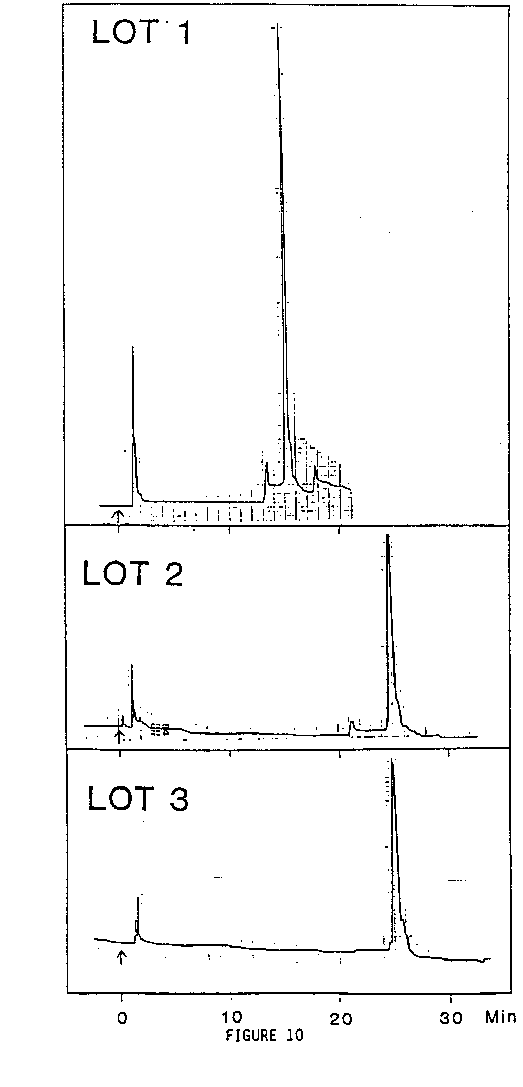

The amino acid sequence of the material isolated by preparative HPLC was found to be correct, including the substitution of Leu for Met at position 27. Samples with a purity of greater than 95% (Figures 9 and 10) were shown to stimulate both adenalyate cyclase in an in vitro assay using bovine pituitary cell extracts and growth hormone release from pituitary cells in culture.

The porcine analog of HuGRF (PGRF) as isolated from pig hypothalami differs in amino acid sequence from HuGRF at three positions towards the carboxy terminus: Ser-34 → Arg; Arg-38 → Gln; Ala-42 → Val (Bohlen et al., Biochem. Biophys. Res. Comm., 116, 726-734 (1983)). Expression of the PGRF$_{Leu27}$ analog of PGRF as a fusion to the TrpLE protein was therefore achieved by specifically deleting the later portion of the LE·BssH II-HuGRF$_{Leu27}$ coding region using restriction endonucleases, and substituting in its place a synthetic DNA fragment coding for the carboxy terminus of PGRF. As in the case of the HuGRF$_{Leu27}$ hormone, the LE·BssH II-PGRF$_{Leu27}$ fusion protein was produced in high yield from induced cells, quantitatively sedimented, and PGRF$_{Leu27}$ easily purified by the identical procedure used for the HuGRF$_{Leu27}$. Material isolated by preparative HPLC was shown to be pure by analysis of the amino acid composition and biologically active by the same criteria applied to HuGRF$_{Leu27}$ above.

The bovine analog of HuGRF (BGRF), as isolated from bovine hypothalami differs from the HuGRF in five positions towards the carboxy terminus: Ser-28 → Asn; Ser-34 → Arg; Arg-38 → Gln; Arg-41 → Lys; Ala-42 → Val. In an analogous manner to the construction and expression of the PGRF$_{Leu27}$, a clone encoding the LE•BssH II-BGRF$_{Leu27}$ polypeptide was constructed, expressed and the BGRF$_{Leu27}$ successfully purified and shown to be pure and biologically active.

The same procedure was thereby successfully applied for expression of the LE•BssH II-PGRF$_{Leu27}$ fusion polypeptide and purification of the GRF$_{Leu27}$ from three different sources, human, porcine and bovine.

More generally, the invention provides a process for preparing the compounds of the invention, which process comprises cleaving a fusion protein to release the compound; or removing a protecting group(s) or solid support from a corresponding protected compound or from a corresponding compound bound to a solid support; or coupling together two fragments of the compound; and thereafter if desired converting a free acid compound into an amide compound, or an amide compound into a free acid compound.

The recombinant DNA process has been described above.

The compounds may also be made by conventional solid phase, or solution, peptide synthesis techniques which are well known in the art.

In general, such synthesis involves the sequential addition of one or more amino acids to a growing peptide chain. In solid phase synthesis, the growing peptide chain is attached to an inert solid support, and at the conclusion of the synthesis the support and any remaining protecting groups are removed.

Free acid, and amide, compounds of the invention are chemically interchanged by conventional techniques.

Standard peptide synthetic techniques such as these are described in numerous places in the literature. Exemplary are U.S. Patent No. 4,529,595 and European Patent Application No. 0145258.

## UTILITY AND ADMINISTRATION

GRFs stimulate the release of endogenous growth hormone from the pituitary, and so raise the plasma concentration of growth hormone. As a result, weight gain is increased in animals and milk yield is improved in lactating mammals.

$BGRF_{Leu27}$ is administered systemically, preferably by intramuscular or subcutaneous injection, or by continuous release through use of an implantable sustained release device.

$BGRF_{Leu27}$ is administered by injection using a pharmaceutically acceptable vehicle, for example physiological saline. Other pharmaceutically acceptable excipients may be added if desired.

$BGRF_{Leu27}$ is administered at a dosage of about 0.01 to about 1.0 µg per Kg body weight per day, preferably about 0.1 to about 0.5 µg/Kg/day. The daily dose is preferably divided into two to four injections per day.

Preferably, $BGRF_{Leu27}$ is dissolved or suspended in physiological saline and the solution or suspension installed in a controlled release device for continuous administration. A typical continuous release rate is from about 1 to about 5 µg per hour per Kg body weight.

87131                                                        25190-FF

## EXAMPLES AND PREPARATIONS

The descriptions in the following examples and preparations will be clear to those skilled in the art of molecular biology or biochemistry.  More detailed descriptions of generally used techniques for nucleic acid manipulation such as treatment with DNA polynucleotide kinase or DNA ligase, separation by electrophoresis, ethanol precipitation, electroelution, and other methods may be found in commonly used texts. See, for example, "Molecular Cloning, A Laboratory Manual" by T. Maniatis, et al., Cold Spring Harbor Laboratory (1982).

The cleavage of DNA by restriction enzyme digestion was carried out according to the current recommendations of the commercial suppliers.

## PREPARATION 1

Parental Plasmids

The plasmid pBR322 is about 4,400 nucleotide bases (4.4 Kb) in length and carries genes for resistance to the antibiotics ampicillin (Amp) and tetracycline (Tet). Its derivation and characterization have been described (F. Bolivar et al., Gene, 2, 95-113 (1977); Locus pBR322 GENBANK, DNA Database, Release 34 (1985)).  The plasmids pUC8 and pUC9 each contain a copy of the Lac promoter and the amino terminus of the gene coding for the enzyme β-galactosidase.  Into the amino terminal region of this gene is inserted a "cassette" comprising a DNA sequence recognized by several useful restriction enzymes.  The plasmid pUC8 and pUC9 differ only in the orientation of this "cassette" and thereby facilitate the manipulation of DNA fragments during cloning.  A description of the construction and characterization of the pUC plasmids has been published (J. Vieira and J. Messing, Gene, 19, 259-268 (1982)).

87131                                                                25190-FF

The E. coli Trp operon, the entire nucleotide sequence of which has been determined and analyzed (C. Yanofsky, et al., Nucleic Acids Res., 9, 6647-6668 (1981); Locus ECOTRP, GENBANK, DNA Database, Release 34 (1985)), was the source of the Trp promoter DNA fragment utilized. The particular plasmid employed was pNO342 which contains a 0.45 Kb fragment of E. coli Trp operon DNA from the closest Pvu II site upstream of the Trp promoter to the first Hinf I site, located in the TrpE gene. To construct pNO342, the Hinf I site on the Trp DNA was filled in and both ends (the Pvu II end and the filled-in Hinf I) then ligated to a Hind III ten base pair-long linker before insertion of the modified DNA fragment into the Hind III site of pBR322. The Hind III site nearest the EcoR I site preceeding the Trp promoter of the pBR322 parent was then destroyed by cutting with Hind III, filling in the ends and religating. The Trp operon DNA could thus be removed from the resultant plasmid by digestion with EcoR I and Hind III. This final construct, pNO342, was obtained from Dr. Neil Osheroff, Biochemistry Department, Stanford University, Palo Alto, California 94305 (current address, Dept. of Biochemistry, Vanderbilt University School of Medicine, Nashville, Tennessee 37232, U.S.A.).

The DNA containing the transcription termination sequence utilized in the constructions described herein was derived from plasmid pVV202t, which was purchased from the laboratory of C. Yanofsky, Department of Biological Sciences, Stanford University, Palo Alto, California 94305. A 0.37 Kb BamH I fragment from pVV202t encompasses 325 base pairs from the transcription termination region of the E. coli rpoC gene (C. Squires, et al. Nucleic Acids Res., 9, 6817-6840 (1981); Locus ECORPLRP, bases #11226-11551 GENBANK Database, (October, 1984)). The TrpLE gene was obtained from plasmid pVV1,

which has been previously described (B.P. Nichols, and C. Yanofsky, *Methods in Enzymology*, <u>101</u>, 155-164 (1983)), and was purchased from the laboratory of C. Yanofsky.

PREPARATION 2

Oligonucleotide Synthesis

The protected deoxyribonucleosides were prepared as described by Ti, et al., <u>J. Am. Chem. Soc.</u>, <u>104</u>, 1316 (1982). All oligonucleotides were synthesized by the solid phase phosphite triester approach (M.H. Caruthers, et al., in "Gene Amplification and Analysis" T. Papas, M. Rosenberg, J. Chirikjian, Eds., Elsevier, New York, pp. 1-26 (1983)). Each chain was built up using successive cycles of deprotection and condensation using 20 molar equivalents of 5'-O-N protected deoxynucleoside-3'-O-methyl-N-morpholino phosphoramidites (T. Dorper and E.L. Winnaker, <u>Nucleic Acids Res.</u>, <u>11</u>, 2575 (1983)) activated by tetrazole, oxidation, and blocking of unreacted sites. After completion of synthesis, the methoxy and the N- protecting groups were removed (Caruthers (1983) op. cit.) and the crude dimethoxytrityl oligonucleotide was purified by reversed phase HPLC. After treatment with 80% acetic acid the fully deprotected product was further purified by electrophoresis on a preparative 20% polyacrylamide gel containing 7 M urea. Purified oligonucleotides were labelled using $[\gamma-^{32}P]ATP$ and polynucleotide kinase. The size and purity was then confirmed by electrophoresis on a 20% polyacrylamide/7 M urea gel.

EXAMPLE 1

Preparation of TrpLE plasmid

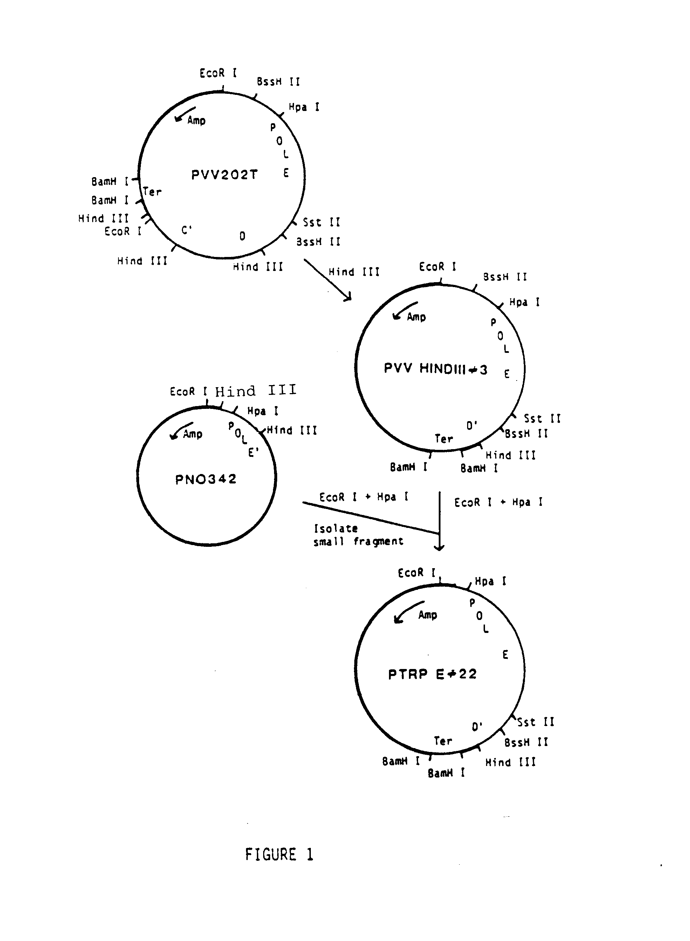

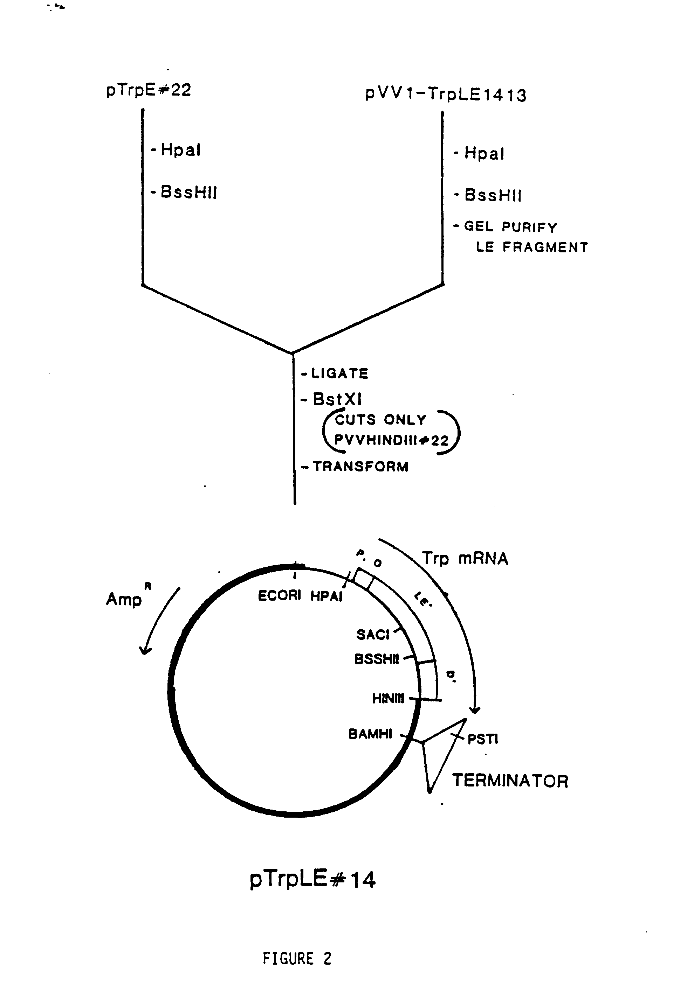

The construction of the plasmid cloning vector pTrpLE-#14 was a multi-step procedure (Figures 1 and 2). In the first step the plasmid pVV2O2t, which contains

0212531

both the Trp E and D genes, as well as a portion of the TrpC gene, was treated to create a unique Hind III site. After digestion of 1.0 µg of pVV2O2t DNA with Hind III, phenol-CHCl3 extraction and ethanol (EtOH) precipitation, 40 ng of the redissolved DNA was self-ligated with T4 DNA ligase and the products transformed into E. coli Hb101. Ampicillin resistant colonies were screened for the presence of a unique Hind III site between the start of the TrpD gene and the terminator; pVV-ΔHind III-#3 had the desired site.

It was desirable to have a plasmid with a unique BssH II restriction site in the TrpE gene for further cloning. Plasmid pVV-ΔHind III-#3 was found to have one or more BssH II restriction sites upstream of the Trp promoter in addition to the BssH II site in the TrpE gene. Consequently, the approximately 2.5 Kb fragment (EcoR I to Hpa I) of pVV-ΔHind III-#3 which contained part of the trp promoter, was replaced with a functionally analogous 282 bp EcoR I to Hpa I fragment from plasmid pNO342. The 0.28 Kb EcoR I to Hpa I fragment from pNO342 was isolated by electroelution from a 5% polyacrylamide gel after digestion of 5 µg of DNA. Then 10 ng of this DNA fragment were ligated with 80 ng of pVV-ΔHind III-#3, which had been previously cleaved with EcoR I and Hpa I. The products were transformed into E. coli Hb101 cells and the ampicillin resistant colonies were screened by digestion with BssH II to identify the desired clone, pTrpE-#22.

Replacement of the Trp promoter-Leader-E gene region of pTrpE-#22 with the analogous portion of pVV1 was the third and final step. DNA from pVV1 (100 µg) was digested with BssH II and Hpa I and electroeluted to produce fragments of about 6.0, 4.0, 1.8, 0.6, 0.57, and 0.54 Kb. The 0.57 Kb fragment containing the TrpLE gene was recovered by electroelution from the 4%

87131                                                    25190-FF

polyacrylamide gel used to separate the digestion products. After phenol-CHCl$_3$ extraction and EtOH precipitation, 0.2 µg of the redissolved 0.57 Kb DNA fragment were ligated with 0.25 µg of the 5.1 Kb DNA fragment of pTrpE-#22, obtained by digestion with BssH II and Hpa I. The ligation products were digested with BstX I, because this recognition site resides in the portion of DNA deleted from the Trp operon in pVV1. Among the ampicillin resistant colonies, pTrpLE-#14 was identified as having the correct structure.

## EXAMPLE 2
### Preparation of a clone coding for TrpLE·Sst II-HuGRF

A synthetic gene coding for HuGRF$_{Leu27}$ was designed to maximize the use of amino acid codons which are favored in highly expressed E. coli proteins, to minimize potential secondary structure in the resultant mRNA, to provide convenient restriction sites for screening the desired clone and to facilitate future alternatives in the DNA sequence. The synthetic gene sequence and the eighteen discrete oligodeoxynucleotides comprising the synthetic gene sequence are shown in the scheme below, and may be referred to in connection with the following more particularized discussion.

To facilitate cloning, the HuGRF$_{Leu27}$ gene was designed to be cloned in two segments; the A segment, comprised of ten oligodeoxynucleotides (A1 through A10), coding for the amino terminal portion of HuGRF$_{Leu27}$, and the B segment, comprised of eight oligodeoxynucleotides, coding for the carboxy terminal portion of the peptide, as illustrated in the following scheme:

```
                            Met Tyr Ala Asp Ala Ile Phe Thr
Sac II                      Nde I
  A1                          A3                        A5
(C)CGC GGG ATG GTC CAT ATG TAC GCT GAT GCT ATA TTC ACT
      CC TAC CAG GTA TAC ATG CGA CTA CGA TAT AAG TGA
   ↑  A2                         A4                     A6
(BssH II compatible)


Asn Ser Tyr Arg Lys Val Leu Glu Gln Leu Ser Ala Arg Lys
A5          A7                  A9                      Hind III
AAC TCT TAC CGC AAG GTT CTG GGC CAA TTA TCA GCA CGC A
TTG AGA ATG GCG TTC CAA GAC CCG GTT AAT AGT CGT GCG TTC GA
A6                      A8                  A10


    Leu Leu Gln Asp Ile Leu Ser Arg Gln Gln Gly Glu Ser Asn
B1       Pst I             B3      Sal I            B5
AG CTT CTG CAG GAC ATT CTT AGT CGA CAA CAG GGC GAA TCT AAC
   A GAC GTC CTG TAA GAA TCA GCT GTT GTC CCG CTT AGA TTG
      B2                        B4


Gln Glu Arg Gly Ala Arg Ala Arg Leu STOP
B5          B7                        Bcl I (BamH I
CAG GAG CGT GGC GCC AGA GCC AGA CTG T      compatible)
GTC CTC GCA CCG CGG TCT CGG TCT GAC ACTAG
B6                      B8
```

To assemble the A segment, each of the ten oligodeoxynucleotides (A1-A10) was individually phosphorylated. 50 pm of each of the ten phosphorylated oligodeoxynucleotides were then combined in a single tube. One tenth volume (20 µl) of 3 M sodium acetate (NaOAc) solution and 4 volumes (800 µl) of absolute EtOH were added with mixing. The solution was chilled for 15 minutes at -70°C and then centrifuged at 12,000 x G at 4°C for 15 minutes. The supernatant was removed and the pellet dried in vacuo. The dried pellet was then resuspended in 48 µl of 66 mM Tris-HCl (pH 7.5), 6.6 mM MgCl$_2$, 10 mM dithiothreitol, and 0.1 mM ATP. To anneal the oligonucleotides, the solution was heated to 40°C and then allowed to cool slowly over a 4 hour period to 20°C. 5 units of T4 DNA ligase were then added and the solution incubated for 12 hours at 20°C. The annealing and ligation reaction was repeated, and the ligated mixture phenol extracted and EtOH-precipitated. The dried, precipitated DNA was resuspended in 50 µl of sterile distilled water, and 35 µl of the ligated oligodeoxynucleotides were cleaved with 100 units each of Hind lII and Sst II. The reaction mixture was incubated at 37°C for 4 hours and EtOH-precipitated. The Sst II/Hind III-HuGRF-A gene segment was gel purified from unreacted and partially ligated DNA fragments by preparative electophoresis on a 6.7% polyacrylamide gel. The recovered DNA is referred to herein as the Sst II/Hind III-HuGRF-A gene segment.

Sixty-two ng of the plasmid pTrpE-#22 DNA which had previously been cleaved with BssH II, Hind III and Sst II was ligated along with about 6.7 ng (0.14 pm) of the Sst II-HuGRF-A gene segment in a final volume of 20 µl with 1.0 units T4 DNA ligase in 66 mM Tris-HCl (pH 7.5), 6.6 mM $MgCl_2$, 10 mM dithiothreitol, 0.1 mM ATP, for 16 hours at 12°C. The ligated DNA mixture was used for transformation of E. coli strain Hbl01. Transformants were selected for ampicillin resistance on L broth plates containing 50 µg/ml ampicillin. After screening 24 independently isolated colonies, eight were found to contain plasmid DNA with the additional Nde I site from the insert as well as the expected Sst II to Hind III insert of about 76 bp. Base sequence analysis according to Maxam and Gilbert verified that one of these had the expected sequence. This plasmid was designated pTrpE·Sst II-HuGRF$_{Leu27}$-A.

To assemble the remainder of the HuGRF$_{Leu27}$ gene, six of the eight oligodeoxynucleotides comprising the B segment (B2-B7) were individually phosphorylated. Phosphorylations were terminated by heating to 70°C for 10 minutes. Oligodeoxynucleotides B1 and B8 were not phosphorylated initially in order to prevent self-ligation at the termini. Two hundred picomoles of each of the phosphorylated oligodeoxynucleotides (B2-B7) were combined with 200 pm each of unphosphorylated B1 and B8. The oligomers were precipitated, annealed, and ligated as described above. The HuGRF-B gene segment was purified from unreacted and partially ligated DNA fragments by preparative electrophoresis on a 7.5% polyacrylamide gel. The 5' ends of the recovered DNA were phosphorylated.

For the assembly of the A and B gene segments of HuGRF$_{Leu27}$, five micrograms of plasmid pTrpE·Sst lI-HuGRF$_{Leu27}$-A was partially digested with 5 units of the restriction endonuclease BamH I. The reaction was terminated by phenol extraction and the DNA EtOH precipitated. The recovered DNA was digested to completion with ten units of the restriction endonuclease Hind III. The mixture was electrophoresed in a 0.7% agarose gel in order to resolve the partial cleavage products. The largest (6.216 Kb) fragment was excised and electroeluted. The recovered DNA was phenol extracted twice and EtOH precipitated.

Sixty ng of the 6.2 Kb Hind III and BamH I- cleaved pTrpE·Sst II-HuGRF$_{Leu27}$-A vector DNA was ligated along with an estimated five fold molar excess of the 72 bp HuGRF-B gene segment. The ligated DNA mixture was used for transformation of E. coli strain Hb101. Tranformants were selected for ampicillin resistance on L broth plates containing 50 µg/ml ampicillin. A number of the transformants were found to contain plasmid DNA with a Sst II to BamH I insert of about 0.5 Kb (the insert/terminator junction does not reconstitute a BamH I site). Base sequence analysis according to Maxam and Gilbert, Proc. Nat. Acad. Sci. USA, 74, 560 (1977), revealed that one of the plasmids, designated pTrpE·Sst II-HuGRF$_{Leu27}$ had the desired HuGRF$_{Leu27}$ coding sequence.

Transfer of HuGRF(Leu27) to pTrpLE-#14

The 0.5 Kb Sst II to BamH I insert comprised of the assembled A and B gene segments of HuGRF$_{Leu27}$ in addition to the transcription terminator, was purified from pTrpE·Sst II-HuGRF$_{Leu27}$ DNA by preparative electrophoresis on a 5% polyacrylamide gel.

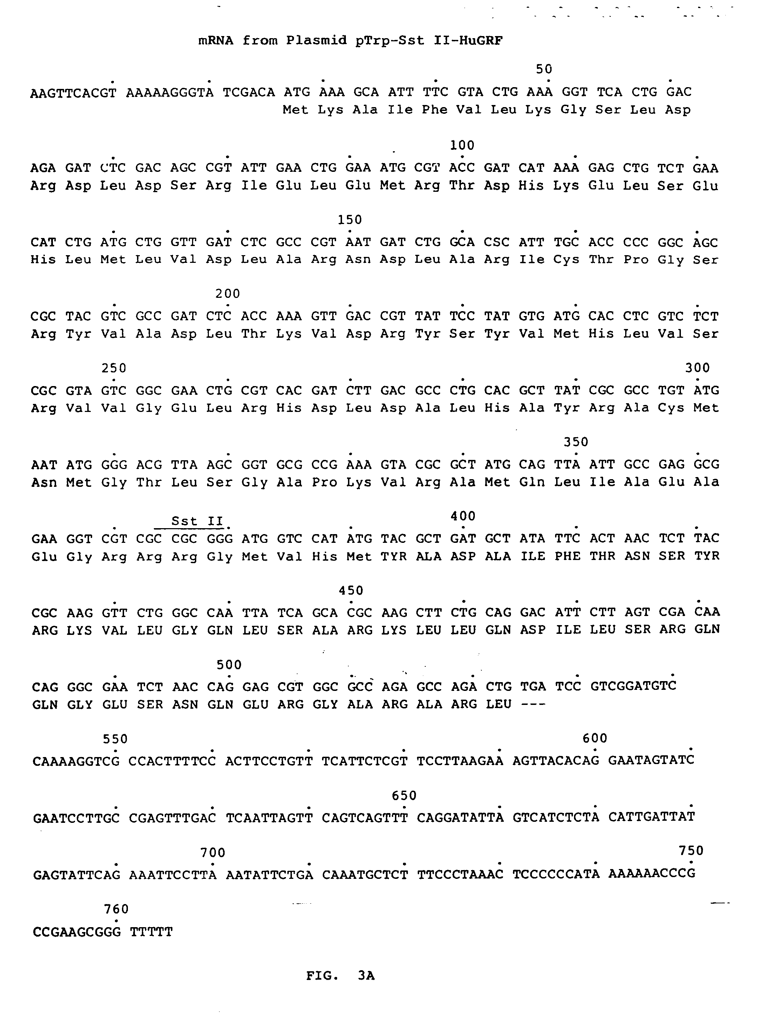

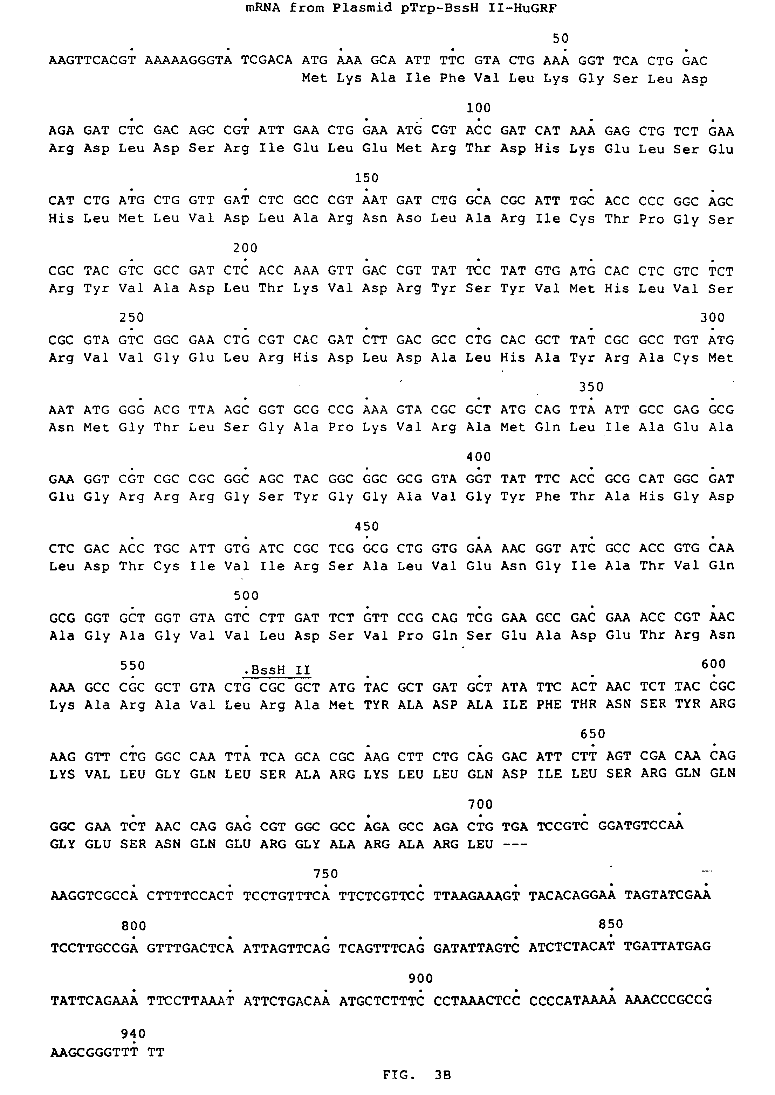

60 ng of BssH II, BamH I and Sst II- cleaved pTrpLE-#14 plasmid DNA was ligated with about a five fold molar excess of the 0.5 Kb Sst II to BamH I fragment. The ligated mixture was used for transformation of E. coli strain Hb101. Transformants were selected for ampicillin resistance on L broth plates containing 50µg/ml ampicillin. After screening 12 independently isolated colonies, 2 were found to contain plasmid DNA with the correct Sst II to BamH I fragment. Base sequence analysis according to Maxam and Gilbert, Proc. Nat. Acad. Sci. U.S.A., 74, 560 (1977), verified that the plasmid pTrpLE·Sst II-HuGRF$_{Leu27}$ had the desired sequence (Figure 3A).

## EXAMPLE 3

Construction of pTrpLE·BssH II-HuGRF(Leu27)

Similarly, one may construct a gene to express TrpLE-HuGRF$_{Leu27}$ using the unique BssH II site rather than the Sst II site in the TrpLE gene.

Eight of the ten oligodeoxynucleotides comprising the A segment of HuGRF$_{Leu27}$ (A2 through A9) were individually phosphorylated. The reaction was terminated by heating to 70°C for 10 min. Oligodeoxynucleotides A1 and A10 were not initially phosphorylated to prevent self-ligation at the termini. 200 picomoles (pM) of each of the phosphorylated oligodeoxynucleotides (A2-A9) were combined with 200 pM of each of A1 and A10. The

87131                                                    25190-FF

oligomers were EtOH precipitated, annealed and ligated. The HuGRF-A gene segment was purified from unreacted and partially ligated DNA fragments by preparative electrophoresis on a 7.5% polyacrylamide gel. The 5' ends of the recovered HuGRF-A segment are then phosphorylated and ligated to plasmid pTrpE-#22, previously cleaved with BssH II and Hind III. The ligation mixture was used to transform E. coli Hbl01. Transformants were screened for the presence of an additional Nde I restriction site. The desired clone was designated pTrpE·BssH II-HuGRF-A.

To complete the HuGRF$_{Leu27}$ gene sequence, the HuGRF-B gene segment as well as the transcriptional terminator were utilized from pTrpE·Sst II-HuGRF$_{Leu27}$. Plasmid pTrpE·Sst II-HuGRF$_{Leu27}$ was cleaved at the unique BamH I and Hind III sites. The 0.4 Kb Hind III to BamH I fragment was purified by gel electrophoresis. Plasmid pTrpE·BssH II-HuGRF-A was cleaved with BamH I and Hind III and the largest fragment purified from a 0.7% agarose gel after electrophoresis. These two purified Hind III to BamH I fragments were ligated and transformed into E. coli Hbl01. Plasmid DNA from the transformants was screened for the presence of 3 Pst I sites (one Pst I site is conferred by the insert). By this technique plasmid pTrpE·BssH II-HuGRF$_{Leu27}$ was identified. Base sequence analysis (Maxam and Gilbert) revealed an unexpected single base pair deletion at the start of the BssH II-HuGRF-A gene segment. Thus, the sequence GCG CGA TG--- was found rather than the expected sequence GCG CGG ATG--. This resulted in the alteration of the triplet reading frame for the remainder of the HuGRF$_{Leu27}$ gene, which was repaired in the next step.

The replacement of the TrpE DNA segment of plasmid pTrpE·BssH II-HuGRF$_{Leu27}$ by the TrpLE DNA segment was achieved by joining portions of two previously described plamids via a synthetic oligonucleotide. This oligonucleotide was designed to replace the HuGRF-A DNA sequence between the BssH II and Nde I sites in order to restore the proper translational reading frame for HuGRF$_{Leu27}$ synthesis. To provide the heterologous HuGRF$_{Leu27}$ coding gene, 12.5 µg of pTrpE·BssH II-HuGRF$_{Leu27}$ DNA was cleaved with Nde I and BamH I and the desired 0.5 Kb fragment isolated from an agarose gel. Next, the self-complimentary synthetic oligonucleotide TAGCGCGC was treated with T4 kinase, and 300 ng was ligated to 0.5 ng of the isolated fragment. This reaction mixture was next heated to 60°C for 15 minutes to inactivate the kinase, and then cleaved with BssH II to convert the Nde I end of the 0.5 Kb Nde I-BamH I fragment into a BssH II site, as illustrated below:

```
                                  Nde I                    BamH I
                                    ↓                         ↓
                  GCG CG(G) ATG GTC CAT ATG TAC----GGATCC---
                  CGC GC(C) TAC CAG GTA TAC ATG----CCTAGG---
                                      I←    0.5 Kb      →I
                                      ↓
                                      + BamH I
                                      + Nde I
                                      ↓
                      TATGTAC-----------G
                      ACATG-----------CCTAG
                                      I
    TAGCGCGC                          I
      CGCGCGAT                        I
        + T₄ Kinase                   I
        ↓                             I
        ↓←---------------------------
        ↓
    TAGCGCGCTATGTAC---------------G
      CGCGCGATACATG---------------CCTAG
      ↓
    + BssH II
      ↓
    CGCGCTATGTAC-------------G
      GATACATG-------------CCTAG
```

The TrpLE coding gene was provided by digestion of plasmid pTrpLE-#14 with BssH II and BamH I. A ligation between 100 ng of the 0.5 Kb BssH II-BamH I fragment and 100 ng of the double digested pTrpLE-#14 DNA was performed. The ligation products were transformed into competent E. coli Hbl01 cells and plated onto ampicillin plates. Restriction enzyme screening of DNA from the resulting colonies by digestion with BssH II plus BamH I or with Pst I identified 7 out of 24 candidates as having the anticipated structure. The DNA sequence of plasmid pTrpLE·BssH II-HuGRF$_{Leu27}$-#15 was confirmed by DNA sequence analysis (Figure 3B).

The procedures described in Examples 6-8 were used to grow E. coli cells (strain W3110 prototroph, ATCC No. 27325) containing plasmid pTrpLE·BssH II-HuGRF$_{Leu27}$#15, express the fusion protein, purify the HuGRF$_{Leu27}$ released by CNBr cleavage and test the biological activity. Results demonstrated activity comparable to BGRF$_{Leu27}$ and PGRF$_{Leu27}$.

## EXAMPLE 4

## Construction And Use Of pTrpLE-PGRF(Leu27)

Using an approach and methods similar to that described in Examples 2-3, plasmids were constructed in which either the unique BssH II site or the unique Sst II site of the pTrpLE-#14 vector was utilized to encode for a fusion protein between the TrpLE protein and the PGRF$_{Leu27}$ analogue of PGRF. This was accomplished by replacing that portion of the synthetic DNA encoding HuGRF located between the Sal I site within the HuGRF DNA segment and the BamH I site at the proximal end of the terminator DNA segment by synthetic DNA encoding PGRF. The replacement of the HuGRF synthetic DNA by the PGRF synthetic DNA was facilitated by first constructing plasmid pPS#8 in which the Sal I site within the HuGRF

87131                                                      25190-FF

gene and the BamH I site just beyond its end were both unique.

## Construction of vector plasmid pPS#8

The BamH I site in plasmid pTrpE-#22 which lies the farthest from the TrpLE gene was first removed by digesting the plasmid with BamH I and Sal I, EtOH precipitating the DNA, and treating the mixture with T4 ligase. (BamH I cuts at each end of the terminator DNA segment, and Sal I cuts within the pBR322 DNA region and within the terminator, nearest the Hind III site.) The ligation products were treated with Sph I (which cuts within the pBR322 DNA region) and transformed into competent Hb101 cells. One of the resulting plasmids, pVVδHinδSal#15, was shown to contain the 0.33 Kb BamH I to Sal I fragment from the transcriptional terminator region of pTrpE-#22 flanked by unique BamH I and Sal I sites. This fragment was in the opposite orientation in pVVδHinδSal#15 compared to pTrpE-#22, but it functions effectively in both orientations.

The Sal I site in pVVδHinδSal#15 was next removed by deleting the DNA between the Sal I and the Pvu II sites of the pBR322 portion of the vector. This was accomplished by digesting pVVδHinδSal#15 with Sal I and Pvu II and filling in the Sal I end by incubating with DNA polymerase I Klenow fragment. The resulting DNA was then cleaved with Sal I and transformed into Hb101 cells. The resulting plasmid, pPS#3, contained the expected deletion and lacked a Sal I site. Since the procedure described would be expected to regenerate a Sal I site, the lack of a Sal I site in pPS#3 probably reflects the increased transformation efficiency of circular DNA lacking a Sal I site, as compared with Sal I-cleaved linear DNA.

Next, the 1.35 Kb EcoR I to BamH I fragment encoding TrpLE-HuGRF$_{Leu27}$ was excised from pTrpLE•BssH II-

8713I                                         25190-FF

HuGRF#5 and ligated with EcoR I and BamH I cleaved pPS#3 DNA. The ligation mixture was treated with BstX I, which cleaves only the pPS#3 DNA fragment not desired in the final construct, and transformed into competent Hb101 cells. The resulting plasmid, pPS#8, containing a gene encoding the TrpLE-HuGRF protein, with its Sal I site, followed by a transcriptional terminator DNA segment, also containing a Sal I site, followed by a unique BamH I site, followed by a transcriptional terminator DNA segment lacking a Sal I site.

Insertion Of Synthetic DNA Encoding PGRF

The three amino acid substitutions which distinguish PGRF from HuGRF all lie beyond the Sal I site in the HuGRF$_{Leu27}$ gene sequence of pPS#8. Since the sequence beyond the BamH I site of pPS#8 contained a functional transcriptional terminator, synthetic DNA encoding PGRF was designed to replace the DNA between these two sites in pPS#8. Six oligonucleotides were synthesized, phosphorylated, annealed, ligated, cleaved with Sal I and BamH I, and the 54 bp oligonucleotide illustrated below isolated from a 5% polyacrylamide gel:

```
        Arg Gln Gln Gly Glu Arg Asn Gln Glu Gln Gly Ala Arg
   C1                          C3                      C5
   _____
    T CGA CAA CAG GGC GCC CGT AAC CAG GAG CAG GGC GCC AGG
   (Sal I) GTT GTC CCG CGG GCA TTG GTC CTC GTC CCG CGG TCC
        C2                          C4
```

```
   Val Arg Leu STOP
   C5          Xba I
   _____
   GTG CGG CTC TAG AG    (BamH I)
   CAC GCC GAG ATC TCC TAG
     C6
```

This 54 bp fragment was ligated to pPS#8 which had been previously cleaved with Sal I and BamH I and the resultant clones screened for the presence of the unique Xba I site which was designed into the synthetic PGRF DNA. Plasmid pTrpLE·BssH II-PGRF#10 was thus identified, followed by experimental verification of the DNA sequence.

By using a similar approach with a vector analogous to pPS#8, but containing a HuGRF gene fused to the Sac II site of TrpLE, the plasmid pTrpLE·Sac II-PGRF was constructed.

Purification and Biological Activity of PGRF(Leu27)

Using the procedures described in Examples 6-8, E. coli cells (strain W3110 prototroph, ATCC No. 27325) containing plasmids encoding the TrpLE-PGRF$_{Leu27}$ fusion protein were grown, expression of the fusion protein induced, and the PGRF$_{Leu27}$ released by CNBr treatment and purified. The purity and biological activity of the final product were comparable to that described for BGRF$_{Leu27}$.

EXAMPLE 5

Construction and Use of a pTrpLE-BGRF(Leu27) Vector

Following the approach outlined above, the carboxy terminal encoding region of the HuGRF portion of plasmid pPS#8 was altered to encode the BGRF$_{Leu27}$ analog of BGRF. Since the five amino acid differences which distinguish BGRF from HuGRF$_{Leu27}$ all lie beyond the Hind III site in the HuGRF$_{Leu27}$ coding region of plasmid pPS#8, eight oligonucleotides were designed to encode the BGRF amino acid sequence and replace the DNA fragment between Hind III and BamH I.

One hundred picomoles of each of the eight oligonucleotides were individually phosphorylated with T4 polynucleotide kinase and ATP. Phosphorylations were terminated by phenol extraction followed by ethanol precipitation with four volumes of ethanol. Fifty picomoles of each of the eight phosphorylated oligonucleotides were then combined in a single tube in a

87131                                                    25190-FF

total volume of 48 μl of ligase buffer and annealed by heating to 42°C followed by cooling slowly to room temperature. Five units of T4 DNA ligase were added and the mixture incubated at room temperature overnight. The annealing procedure was then repeated as before, another 5 units of ligase added, and the mixture again incubated for 16 hours at room temperature. The ligation mixture was then phenol extracted and ethanol precipitated. The dried pellet was resuspended in 300 μl Hind III buffer and digested with 200 units Hind III for 4 hours at 37°C. Following ethanol precipitation, the dried pellet was resuspended in 300 μl of Bam HI buffer and digested with 200 units of Bam HI for 4 hours at 37°C. The mixture was then phenol extracted and ethanol precipitated with four volumes of ethanol. After electrophoresis on a 6.7% polyacrylamide gel, the expected approximately 76 b.p. Hind III to Bam HI fragment was excised from the gel and electroeluted. The recovered DNA was ethanol precipitated and the dried pellet resuspended in 20 μl of distilled water.

A 1 μl aliquot of the recovered approximately 76 b.p. Hind III-Bam HI fragment was ligated to 100 ng of Bam HI and Hind III digested puc8 vector in a 20 μl reaction mixture for 24 hours at room temperature. This ligation mixture was transformed into competent E. coli JM 103 cells and the transformation mixture plated onto L agar plates containing 50 μg/ml ampicillin, 50 μmolar isopropyl β-D-thiogalactopyranoside and 0.005% 5-Bromo-4-Chloro-3-Indolyl β-D-Galactopyranoside. Twelve colorless colonies were randomly picked after growth overnight at 37°C, and plasmid DNA prepared from these clones. Of the twelve isolates, plasmid DNA from three were found to contain a unique Xba I site, a unique Pst I site and an approximately 76 b.p. Hind III to Bam HI insert fragment. Upon DNA sequence analysis, two of these plasmid DNA preparations were found to have the desired DNA sequence. The 76 b.p. Hind III to Bam HI fragment:

```
          Leu Leu Gln Asp Ile Leu Asn Arg Gln Gln Gly Glu Arg
       D1                          D3                        D5
   5' AG CTT CTG CAG GAC ATT CTT AAC CGA CAA CAG GGC GAA CGT
   3'    A GAC GTC CTG TAA GAA TTG GCT GTT GTC CCG CTT GCA
Hind III D2                                      D4
```

```
   Asn Gln Glu Gln Gly Ala Lys Val Arg Leu STOP
   D5                  D7                            BamH I
   AAC CAG GAG CAG GGC GCC AAG GTG CGG CTC TAG AG
   TTG GTC CTC GTC CCG CGG TTC CAC GCC GAG ATC TCC TAG
        D6                      D8
```

was removed from one of these plasmid DNA's and ligated into Hind III and Bam HI cleaved plasmid pPS#8 to produce the desired plasmid, pTrpLE BssH II-BGRF$_{Leu27}$#15.

## EXAMPLE 6
### Expression and Purification of LE-BGRF(Leu27)

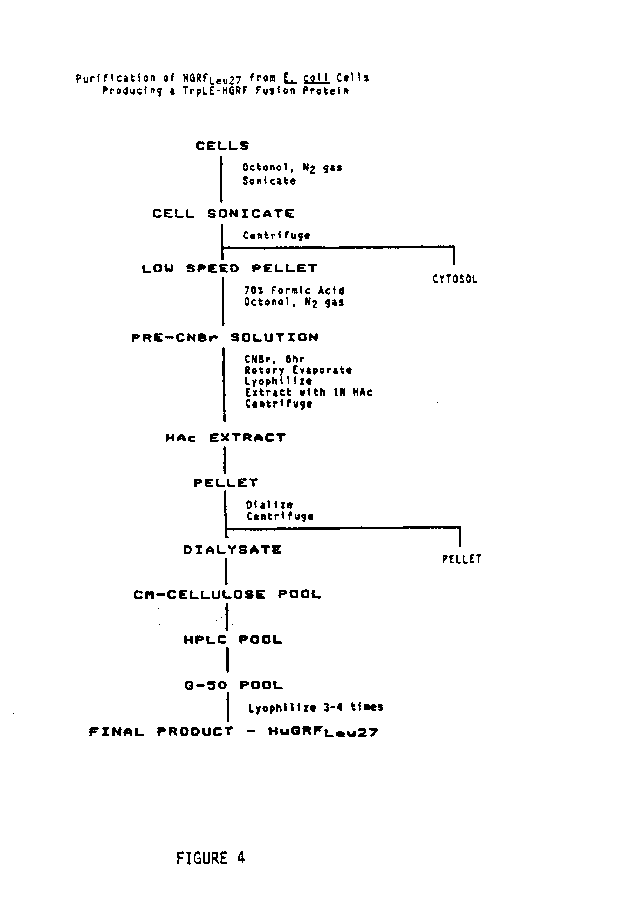

The procedure described herein can be used to purify GRF from LE-GRF$_{Leu27}$ clones (producing HuGRF$_{Leu27}$, BGRF$_{Leu27}$, or PGRF$_{Leu27}$) which utilize either the BssH II or Sac II fusion site to the TrpLE protein. Before beginning, the extent of induction can be monitored by analysis of an aliquot of the washed cell pellet on a stained SDS gel. There is no difference in the level of expression or the purification steps up to the point of dialysis with either clone (Fig. 4). During dialysis, the BssH II clone deposits a large precipitate, whereas the Sac II clone forms a smaller precipitate. Centrifugation of the BssH II dialysate thus leaves a larger pellet which must be thorougly washed to fully recover the LE-GRF$_{Leu27}$.

### Induction of TrpLE-BGRF

In this case, a starter culture of 50 ml of LBr culture media (reference to the preparation of the LBr and M9 media described here can be found in J. H. Miller (1972) "Experiments in Molecular Genetics," p. 431) containing 50 µg/ml of ampicillin and 100 µg/ml of tryptophan is innoculated with E. coli cells containing the plasmid pTrpLE·BssH II-BGRF$_{Leu27}$, and grown overnight at 37°C with vigorous shaking to a final A$_{550}$ of about 6. Four liters of M9 culture media for production are pre-warmed to 37°C and seeded with 40 ml of the LBr starter culture to give a final A$_{550}$ of about 0.6. The culture is then grown with vigorous shaking to an A$_{550}$ of about 0.2 to 0.5, whereupon 4 ml of a 10 mg/ml solution of indole acrylic acid (IAA) in EtOH is added to give a final concentration of 10 µg/ml solution of IAA. Growth is then continued with good aeration for about 16 hr to a final A$_{550}$ of about 5 (typically, about 5 to 10). The addition of IAA during growth in M9 media is optional since it increases only the kinetics of product accumulation and not the final yield. An alternate and equally effective method of cell growth is to substitute LBr for M9, in which case the addition of IAA is essential for adequate product yields. The cells are concentrated by centrifugation, resuspended in 500 ml (typically 1/10 to 1/20 of the original culture volume) of 50 mM Tris-HCl, pH 7.1, and re-pelleted. The washed cells may be conveniently stored at -20°C at this stage.

Sonication

The washed cells are lysed by sonic disruption. Cells are resuspended in 900 ml (typically 20-100 A$_{550}$ units per ml) of 50 mM Tris-HCl, pH 7.5, 0.1 mM EDTA, 1 mM phenylmethylsulfonyl fluoride (PMSF) (this buffer solution is abbreviated "TE-PMSF"), cooled to 4°C, a few drops of octanol added and N$_2$ gas bubbled through the solution. These steps serve to minimize heating of the sample and oxidation of methionine residues during sonication. The suspension is sonicated using a model W220F Heat Systems-Ultrasonics, Inc. sonicator (equipped with a 3/4" horn) according to the manufacturer's recommendations. The soncicator is typically operated only 50% of the total process time to avoid overheating.

Centrifugation

The cell lysate is centrifuged for 15 min at about $3600 \times g_{max}$ to pellet the pTrpLE-BGRF$_{Leu27}$ and the supernatant discarded. The pellet is resuspended in 450 ml TE-PMSF (typically 40-80 A$_{550}$/ml) and the pellet collected by centrifugation as before.

EXAMPLE 7

Purification of BGRF(Leu27)

Cleavage of the methionine residues in the TrpLE-BGRF$_{Leu27}$ fusion protein with CNBr causes release of the desired heterologous BGRF$_{Leu27}$ protein, in the -OH form, which can be purified as described below.

CNBr Treatment of Pellet

The washed pellet of TrpLE-BGRF$_{Leu27}$ is resuspended by gently stirring or swirling it in 60 ml 70% formic acid (about 20 mg/ml total protein; typically material from 1000 A$_{550}$ units of cells is dissolved in 3 ml). A few drops of octanol are added and N$_2$ bubbled through the solution for 20 minutes before adding 5.5 g CNBr. This reaction is allowed to proceed for 6 hours at 25°C before adding an equal volume of 50:50 MeOH:H$_2$O in order to facilitate the subsequent concentration step. The volatile excess CNBr, water and organic solvents are removed by rotary evaporation until the volume is reduced by one-half. Additional 50:50 MeOH:H$_2$O is mixed with the sample and subsequently removed by rotary evaporation. After 2-4 repetitions of this process, the bulk of the formic acid and CNBr will have been removed.

87131                                                                 25190-FF

The sample is then evaporated to dryness, redissolved in 200 ml of water and lyophilized.

Acetic Acid Extraction

The lyophilized sample is dissolved in 80 ml 1 N acetic acid, and extracted for at least 30 minutes at 25°C. After centrifugation for 10 minutes at 12,000 x $g_{max}$ the supernatant is recovered and the pellet reextracted with 70 ml 1 N acetic acid as before. The pellet fraction is discarded and the supernatants, which contain the $BGRF_{Leu27}$, are combined.

Dialysis

The 1 N HOAc extract is next placed into dialysis tubing having a molecular weight cutoff of 3500 and dialyzed for a total of 18 h against four changes of 10 mM NaOAc, pH 5.0, 4 L each. The first half of the dialysis is performed at 25°C, and the second half at 4°C. The precipitate which forms is removed by centrifugation for 10 min. at 10,000 x $g_{max}$. If warranted, the pellet is washed with buffer to maximize recovery of BGRF in the soluble fraction.

CM-Cellulose Chromatography

The clarified dialysate is then passed through a 50 ml bed volume CM-cellulose column (2.5x10 cm) at 1.5 ml/min in a cold room (0-4°C). After washing with 50 mM NaOAc, pH 5, a gradient from 50 to 500 mM NaOAc, pH 5 is used to elute the $BGRF_{Leu27}$. The peak of $BGRF_{Leu27}$ can be determined by immunoblotting (western analysis) of the column fractions.

HPLC

Pooled fractions from the CM-cellulose column are filtered through a milex-GV 0.22 μm filter (Milipore) and applied at 4 ml/min directly onto an 0.8x25 cm HPLC column packed with Vydec C18 TP201 N-capped (10 μm) 300 Å pore beads which have been equilibrated in solvent "A" (0.1% trifluoroacetic acid in water). The column is then washed with 75:25 solvent A:solvent "B" (0.1% trifluoroacetic acid in acetonitrile) until the baseline returns to zero. The $BGRF_{Leu27}$ is then eluted with a gradient running from 25 to 35% solvent B over a period of 30 min. The major peak in the chromatogram, which contains the BGRF, elutes after about 26 minutes and is collected. The solvent is removed by 3-4 cycles of rotory evaporation from water.

Gel Filtration

As a final purification step, and in order to replace any remaining traces of $CF_3CO_2-$ with Cl-, the dried sample is dissolved in 4 ml of 25 mM HCl and applied to a 25 ml column (2.5x5cm) of Sephadex G-50 which has been equilibrated in the solvent. The column effluent is monitored by absorbance at 280 nm and the void volume peak containing the $BGRF_{Leu27}$ is pooled. This material is lyophilized several times from water until the pH of the solution exceeds 5. A white, fluffy material is obtained.

## EXAMPLE 8

Characterization of BGRF(Leu27)

The final product was $BGRF_{Leu27}$ in the -OH form ($BGRF_{Leu27}$-OH).

A purity of greater than 95% for this final $BGRF_{Leu27}$ product was demonstrated by SDS gels (Burr and Burr, Meth. Enz., 96, 239-244 (1983)) stained for total protein.

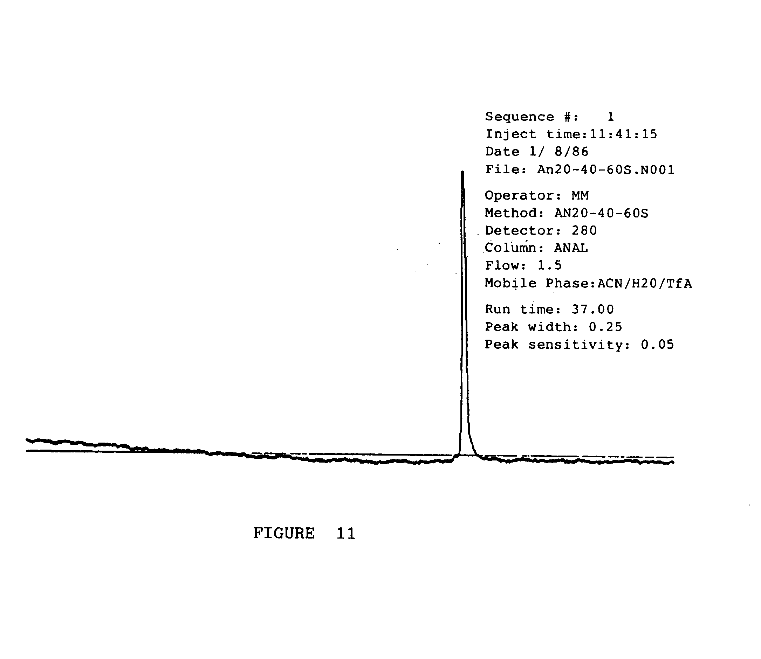

In addition, a reverse phase HPLC profile was obtained (Figure 11).

When the final $BGRF_{Leu27}$ product was subjected to amino acid sequence analysis (Hunkapiller, M.W., Hewick, R.M., Dreyer, W.F. and Hood, L.E., Methods in Enzymology 91, 399-413 (1983)) the predicted result was obtained.

EXAMPLE 9

Biological Activity of BGRF(Leu27)-OH

The 44-amino acid peptide GRF induces secretion of growth hormone from the anterior pitutitary gland. The mechanism of action involves a receptor-mediated activation of adenylate cyclase and elevation of intracellular cyclic AMP (Labrie, F., B. Gagne, and G. Lefevere, Life Sciences, 33, 2229-2223 (1983)). The biological potency of $BGRF_{Leu27}$ is therefore demonstrated by its ability to activate adenylate cyclase, by its specific binding to pituitary cells, and by its ability to stimulate the release of GH from pituitary cells.

Adenylate Cyclase Enzyme Activation Assay

To assay adenylate cyclase activation by GRF we chose either porcine or bovine pituitary glands. Pituitary membranes were lyophilized and enzyme activity was assayed in accordance with established procedures (Alvarez, R., and J.J. Bruno, Proc. Nat. Acad. Sci. USA, 74, 92-95 (1977)).

$BGRF_{Leu27}$ had an $EC_{50}$ of 2 µM.

Binding Assay

The specific binding of GRF to pituitary cells was determined as follows:

GH3 rat pituitary tumor cells, a somatrophic cell line which was obtained from Dr. D. Gospodarowicz (University of California, San Francisco), were grown in Dulbecco's minimal essential medium (DMEM). Synthetic $HuGRF-NH_2$ was purchased from Peninsula Labs (San Carlos, CA) and radioactively labeled with $Na[^{125}I]$ using Enzymobeads (BioRad) to a specific activity of 20,000 cpm/ng protein. Cells were grown to confluence in 24-well Costar trays washed with DMEM buffered at pH 7.4 with 20 mM Hepes and containing 0.1% bovine serum albumin (DMEM-BSA). Cells were incubated at 4°C for one hour

with $2 \times 10^{-9}$ M $[^{125}I]$-HuGRF(1-44) in either the absence or presence of increasing concentrations of recombinant GRF analogs in a final volume of 250 μl DMEM-BSA. Cells were washed three times with DMEM-BSA, lysed with 0.1 N NaOH and the cell-associated radioactivity was determined in a gamma counter. Nonspecific binding was assessed in the presence of $10^{-7}$ M synthetic HuGRF-NH$_2$ and did not exceed 15% of the specific binding.

In independent experiments it was established that GH3 cells possess specific receptors for HuGRF-NH$_2$. Binding was saturable and reversible. Scatchard analysis of this data revealed about 20,000 binding sites per cell with an apparent $K_d$ for GRF-(1-44) of about $1.0 \times 10^9$ $M^{-1}$.

The recombinant bovine GRF$_{Leu27}$ was active in this test.

Growth Hormone Secretion Assay

The ability of GRF to stimulate the release of growth hormone from pituitary cells was also assayed. Normal rat anterior pituitary cells were established in primary cultures as follows: Male adult Sprague-Dawley rats (250 g) were sacrificed by decapitation. The anterior pituitary was dissected, placed in DMEM containing 1% BSA and minced into 1 mm pieces. After several washes with DMEM-BSA, the tissue was incubated at 37°C for 35 minutes with 0.2 U trypsin/ml and 1 mg/ml DNAase/ml with gentle shaking. Tissue fragments were then further dispersed by physical shearing with a Pasteur pipette. Large fragments were allowed to settle and were removed. Cells in the supernantant were then pelleted, washed and plated for tissue culture for 4 days in DMEM + 10% FCS before an experiment. The average yield of viable cells was approximately $2 \times 10^6$ cells per pituitary. Cells were plated in 24-well dishes and incubated with GRF or the test compound for 3 hours at 37°C.

The concentration of GH in the medium was determined by a solid phase RIA using reagents from the NIH Pituitary Agency. Ninety-six well polyvinylchloride plates were coated overnight at 4°C with rabbit anti-monkey Ig antibodies (Pel-Freez) at 0.1 mg/ml in phosphate buffered saline (PBS). Plates were countercoated with a 3% BSA solution in PBS for at least one hour at 37°C. Plates were washed and incubated with monkey anti-rat GH antiserum (1/10,000 final dilution) for 2 hours or more at 37°C. After several washes with PBS-0.1% BSA, plates were incubated at 37°C for 2 hours with 20 ng/ml [$^{125}$I]-GH in the presence or absence of unlabeled GH or dilutions of cellular conditioned medium. After several washes, plates were dried and individual wells were cut out and counted in a gamma counter.

The recombinant bovine $GRF_{Leu27}$ was potent and efficacious for the induction of GH release.

<div align="center">EXAMPLE 10</div>

Biological Activity of BGRF(Leu27)-OH in Cattle and Swine
Cattle.

The capability of BGRF(Leu27) to stimulate growth hormone (GH) secretion in cattle was tested by pulse intravenous injections of the compound. Three Holstein calves were fitted with indwelling jugular catheters. The BGRF(Leu27) was dissolved in .9% NaCl at appropriate concentrations to allow injection volumes of approximately 2 ml. A random sequence of .15 and .45 μg of BGRF(Leu27)/kg of body weight (BW) was given to the calves by once-daily pulse intravenous injections at 0800 h on consecutive days. Each concentration was tested in each calf on two different days. Blood samples for analysis of GH were obtained at -30, -15, 0, 5, 10,

8713I

25190-FF

15 and 20 min and at 20 min intervals throughout 120 min after each injection. Bovine GH was assayed by double antibody radioimmunoassay. Area under the GH-time curve (AUC) was calculated by a trapezoidal summation method. The number of animals responding by an increase in the concentration of GH in plasma, amplitude of the GH peaks, and area under the GH-time curve (AUC) were used to determine the biological activity and potency of the compound.

Pulse injection by BGRF(Leu27) at both .15 and .45 µg of BGRF(Leu27)/kgBW caused release of GH following every injection. GH in plasma increased from a preinjection concentration of 6.9 ng/ml to more than 50 ng/ml within approximately 30 minutes after injection of the .15 and .45 µg/kg doses. AUC values increased in proportion to the dose of BGRF(Leu27).

Table 1. Characteristics of growth hormone in plasma of calves injected with BGRF(Leu27)

| Variable | Dose of BGRF(Leu27) | |
|---|---|---|
| | .15 | .45 |
| Number of responses | 6/6 | 6/6 |
| Time of peaks, min.[a] | 25 | 30 |
| Peak amplitude, ng/ml[a,b] | 60.9 | 56.5 |
| $AUC_{120}$, ng·min/ml[a] | 2125 | 2668 |

[a]Means of animals which responded.
[b]Mean preinjection GH concentration was 6.9 ng/ml.

Swine.

The capability of BGRF(Leu27) to stimulate GH secretion in swine was tested by continuous intravenous infusion of the compound. Four white crossbred female pigs weighing 40 to 44 kg were fitted with two indwelling catheters in opposite jugular veins. The pigs received a continuous intravenous infusion of either saline

87131                                                    25190-FF

(control) or BGRF(Leu27) at the rate of 1 µg/kgBW for seven hours. Blood samples were obtained at 15 minute intervals throughout the infusion period for determination of GH in plasma. The time-series of GH concentrations in plasma was used as a determination of response to GRF.

Infusion of BGRF(Leu27) caused a four-fold increase in the amplitude of the GH peaks observed in plasma during the 7 hours of infusion (Table 2). Likewise, the area of individual peaks was increased approximately 7-fold while the total area under the GH response curve was more than doubled by intravenous infusion of BGRF(Leu27).

Table 2. Growth hormone response in plasma
of swine to infusion of BGRF(Leu27)

| Variable | Control | GRF | SE |
|---|---|---|---|
| Number of observations | 4 | 3 | - |
| Baseline, ng/ml | 5.4 | 8.1 | 1.4 |
| Number of peaks | 1.3 | 1.0 | .4 |
| Peak amplitude, ng/ml | 12.2 | 56.2 | 2.5 |
| Area of peaks, ng·min/ml[a] | 313.0 | 2190.3 | 22.2 |
| Total AUC, ng·h/ml[b] | 44.8 | 100.8 | 7.0 |

[a] Average area of individual peaks above baseline.
[b] Total area under the GH response curve including baseline.

EXAMPLE 11

Formulations

BGRF$_{Leu27}$ is dissolved or suspended in physiological saline at a concentration of about 1 to 5 µg/ml. Other pharmaceutically acceptable excipients may be added if desired. Two ml of the resulting solution is administered by intramuscular or subcutaneous

-43-

injection one to four times per day. A typical mammalian dose is about 0.1 to 0.5 μg per Kg body weight per day.

Alternatively, BGRF$_{Leu27}$ may be dissolved or suspended in physiological saline and the solution or suspension installed in a controlled release device for continuous administration. A typical continuous release rate is from about 1 to about 5 μg per hour per Kg body weight.

## EXAMPLE 12

### Preparation of BGRF(Leu27)-NH$_2$ and BGRF(Leu27)-OH by Solid Phase Synthesis

As noted above, the BGRF(Leu27) prepared by recombinant DNA techniques was isolated in the -OH form, BGRF(Leu27)-OH.

The corresponding -NH$_2$ compound, BGRF(Leu27)-NH$_2$, may conveniently be made by solid phase techniques, as follows.

### (a) Preparation of HuGRF (Leu 27)-NH$_2$

In the reaction vessel of a Beckman 990 Peptide Synthesizer was placed 5.78g of benzhydrylamino-polystyrene-1% divinylbenzene with 0.864 meq amine/g resin loading (Beckman). This was reacted with 3.12g (12.5 mmol) of the first amino acid, Boc-Leu-OH•H$_2$O, in the presence of diisopropyl carbodiimide and 1.7g (12.5 mmol) of 1-hydroxybenzotriazole (HBT) for 4 hr. After the incorporation of the first amino acid, others were added using the standard coupling program using:

4.28g of BOC-Arg(TS)-OH,

2.36g of BOC-Ala-OH,

4.28g of BOC-Arg(TS)-OH,

2.36g of BOC-Ala-OH,

2.19g of BOC-Gly-OH,

4.26g of BOC-Arg(Ts)-OH,

4.2g  of BOC-Glu(OBzl)-OH,

3.08g of BOC-Gln-OH in the presence of 1.7g of HBT,

2.32g of BOC-Asn-OH in the presence of 1.36g of HBT,

4.2g  of BOC-Glu(OBzl)-OH,

2.19g of BOC-Gly-OH,

2.46g of BOC-Gln-OH in the presence of 1.36g of HBT,

2.46g of BOC-Gln-OH in the presence of 1.36g of HBT,

4.28g of BOC-Arg(Ts)-OH,

3.69g of BOC-Ser(Bzl)-OH,

This resulted in 44g of peptide resin containing ~5mmol of the C-terminal 16 residues of the product. It was split and ~33g was carried forward (~3.5mmol scale) by coupling with:

1.74g of BOC-Leu-OH•$H_2O$,

1.68g of BOC-Ile-OH•1/2 $H_2O$,

2.26g of BOC-Asp(OBzl)-OH,

1.72g of BOC-Gln-OH in the presence of 0.95g of HBT,

1.74g of BOC-Leu-OH•$H_2O$,

1.74g of BOC-Leu-OH•$H_2O$,

4.1g  of BOC-Lys(Z-Cl)-OH-DCHA Salt, freed from DCHA by partitioning with $CH_2Cl_2$ and $NaHSO_4$,

3.0g  of BOC-Arg(Ts)-OH,

1.32g of BOC-Ala-OH,

2.07g of BOC-Ser(Bzl)-OH,

1.74g of BOC-Leu-OH•$H_2O$,

1.72g of BOC-Gln-OH in the presence of 0.95g of HBT,

1.23g of BOC-Gly-OH,

1.74g of BOC-Leu-OH•H$_2$O

1.52g of BOC-Val-OH,

4.1g of BOC-Lys(Z-Cl)-OH-DCHA Salt, freed of salt as above,

3.0g of BOC-Arg(Ts)-OH,

3.08g of BOC-Tyr(Bzl-Cl$_2$)-OH,

2.07g of BOC-Ser(Bzl)-OH,

1.62g of BOC-Asn-OH, in the presence of 0.95g of HBT,

2.16g of BOC-Thr(Bzl)-OH

This resulted in ~40g of peptide resin which was split, and ~15g was carried forward by coupling with

0.795g of BOC-Phe-OH,

0.72g of BOC-Ile-OH•1/2H$_2$O

0.57g of BOC-Ala-OH,

0.65g of BOC-Asp(OBzl)

This resulted in ~14g of peptide resin of which ~5g was carried forward by coupling with:

0.2g of BOC-Ala-OH, in the presence of 0.3g of HBT

0.4g of BOC-Tyr(Bzl-Cl$_2$)-OH in the presence of 0.3g of HBT, to result in 2g of the 1-44 protected peptide resin.

A 1g portion of this material was deprotected by treatment with 10ml of 90% HF/anisole at -10° for 1/2 hour and at 0° for 1/2 hr. After removal of HF at 0° the residue was washed with Et$_2$O. The product was dissolved in 10% acetic acid and passed through an AG$_3$ (8Ac form; BioRad) to exchange the counter-ion from F$^-$ to Ac$^-$. The effluent was concentrated and lyophilized to give the impure product as 0.3g of slightly yellow powder.

The product was passed through a Sephadex G-50 column (3x70cm) in 2M acetic acid. Fractions 12-19 were pooled, concentrated and lyophilized to yield 150mg of yellow solid. The partially purified product was submitted to ion exchange chromatography on CM-cellulose (3x70cm) using two gradients from 0.05M $NH_4OAc$ (pH 4.5) to 0.5M $NH_4OAc$ (pH6.5) and from 0.5M $NH_4OAc$ (pH6.5) to 1M $NH_4OAc$ (pH6.5), each gradient vessel began with 500 ml of eluent. Fractions were monitored by high performance liquid chromatography using a Vydac TP-218 $C_{18}$ reversed-phase column and as eluent a gradient from 30 to 40% $CH_3CN$ ($0.1\%CF_3CO_2H$). Fractions 50 and 51 were pooled, concentrated and lyophilized to yield purified [Leu$^{27}$] HuGRF(1-44)-$NH_2$ with $[\alpha 25]_D$-21.9° (C0.2, $H_2O$) and m.p. 194-196°; Amino Acid Analysis: Asp, 3.9(4); Thr, 0.9 (1); Ser 3.7 (4); Glu, 7.2 (7); Gly 3.5 (3); Ala 4.8 (5); Val, 1.2 (1); Ile, 1.6 (2); Leu, 6.0 (6); Tyr, 1.6 (2); Phe, 0.7 (1); Lys, 2.2 (2); Arg, 6.3 (6); $NH_3$ 8.4.

(b) Preparation of BGRF (Leu27)-$NH_2$

BGRF (Leu 27)-$NH_2$ may be made in exactly similar manner to that detailed in (a) above for HuGRF (Leu27)-$NH_2$, differing only in the use of the appropriate amino acids to give the bovine rather than the human structure.

(c) Preparation of BGRF (Leu27)-OH

In similar manner to (b) above, BGRF(Leu27)-OH may be prepared by solid phase synthesis.

CLAIMS

1.    The polypeptide bovine growth hormone releasing factor in which Met27 is replaced with Leu ($BGRF_{Leu27}$),the carboxy end group being in free acid (-OH) or amide ($-NH_2$) form..

2.    A process for preparing a compound of Claim 1, which process comprises cleaving a fusion protein to release the compound; or removing a protecting group(s) or solid support from a corresponding protected compound or from a corresponding compound bound to a solid support; or coupling together two fragments of the compound; and thereafter if desired converting a free acid compound into an amide compound, or an amide compound into a free acid compound.

3.    A process according to Claim 2, which process comprises:
        selecting a plasmid containing a suitable native DNA sequence;
        cleaving the native sequence with a restriction endonuclease;
        inserting a $BGRF_{Leu27}$ DNA sequence within the native sequence in the correct reading frame to produce a plasmid encoding a native polypeptide fragment-$BGRF_{Leu27}$ fusion protein;
        transforming a suitable bacterial host with the resulting fusion protein-encoding plasmid;
        expressing the native polypeptide fragment-$BGRF_{Leu27}$ fusion protein; and
        cleaving the fusion protein to release $BGRF_{Leu27}$.

4.    The process of Claim 3 wherein the suitable native DNA sequence is TrpLE.

5.    The process of Claim 3 or 4 which further comprises inserting a cleavable linker DNA sequence between said native DNA sequence and said $BGRF_{Leu27}$ DNA sequence.

6.    A plasmid expression vector for producing $BGRF_{Leu27}$, which vector comprises:

a plasmid containing a suitable native DNA sequence;

a $BGRF_{Leu27}$ DNA sequence in the same reading frame and continuous with said native DNA sequence.

7.    The vector of Claim 6 which further comprises a cleavable linker DNA sequence between said native DNA sequence and said $BGRF_{Leu27}$ DNA sequence.

8.    The vector of Claim 7 wherein said native DNA sequence is TrpE.

9.    The vector of Claim 7 wherein said native DNA sequence is TrpLE.

10.    A method for increasing weight gain in animals, which method comprises administering to an animal a compound of Claim 1.

11.    The method of Claim 10 wherein said animal is bovine, porcine, or poultry.

12.    A method for increasing milk production in lactating mammals, which method comprises administering to a mammal a compound of Claim 1.

13.    A formulation comprising a compound of Claim 1 and a pharmaceutically acceptable excipient.

14.    A native polypeptide fragment - $BGRF_{Leu27}$ fusion protein.

FIGURE 1

0212531

pTrpE#22                    pVV1-TrpLE1413

- HpaI                      - HpaI

- BssHII                    - BssHII

                            - GEL PURIFY
                              LE FRAGMENT

- LIGATE

- BstXI
  (CUTS ONLY
   PVVHINDIII#22)

- TRANSFORM

Trp mRNA

P. O

ECORI HPAI

L E.

Amp^R

SACI

BSSHII

O.

HINIII

BAMHI — PSTI

TERMINATOR

pTrpLE#14

FIGURE 2

mRNA from Plasmid pTrp-Sst II-HuGRF

                                                              50
AAGTTCACGT AAAAAGGGTA TCGACA ATG AAA GCA ATT TTC GTA CTG AAA GGT TCA CTG GAC
                        Met Lys Ala Ile Phe Val Leu Lys Gly Ser Leu Asp

                                         100
AGA GAT CTC GAC AGC CGT ATT GAA CTG GAA ATG CGT ACC GAT CAT AAA GAG CTG TCT GAA
Arg Asp Leu Asp Ser Arg Ile Glu Leu Glu Met Arg Thr Asp His Lys Glu Leu Ser Glu

                              150
CAT CTG ATG CTG GTT GAT CTC GCC CGT AAT GAT CTG GCA CSC ATT TGC ACC CCC GGC AGC
His Leu Met Leu Val Asp Leu Ala Arg Asn Asp Leu Ala Arg Ile Cys Thr Pro Gly Ser

                    200
CGC TAC GTC GCC GAT CTC ACC AAA GTT GAC CGT TAT TCC TAT GTG ATG CAC CTC GTC TCT
Arg Tyr Val Ala Asp Leu Thr Lys Val Asp Arg Tyr Ser Tyr Val Met His Leu Val Ser

     250                                                             300
CGC GTA GTC GGC GAA CTG CGT CAC GAT CTT GAC GCC CTG CAC GCT TAT CGC GCC TGT ATG
Arg Val Val Gly Glu Leu Arg His Asp Leu Asp Ala Leu His Ala Tyr Arg Ala Cys Met

                                                   350
AAT ATG GGG ACG TTA AGC GGT GCG CCG AAA GTA CGC GCT ATG CAG TTA ATT GCC GAG GCG
Asn Met Gly Thr Leu Ser Gly Ala Pro Lys Val Arg Ala Met Gln Leu Ile Ala Glu Ala

              Sst II                        400
GAA GGT CGT CGC CGC GGG ATG GTC CAT ATG TAC GCT GAT GCT ATA TTC ACT AAC TCT TAC
Glu Gly Arg Arg Arg Gly Met Val His Met TYR ALA ASP ALA ILE PHE THR ASN SER TYR

                         450
CGC AAG GTT CTG GGC CAA TTA TCA GCA CGC AAG CTT CTG CAG GAC ATT CTT AGT CGA CAA
ARG LYS VAL LEU GLY GLN LEU SER ALA ARG LYS LEU LEU GLN ASP ILE LEU SER ARG GLN

              500
CAG GGC GAA TCT AAC CAG GAG CGT GGC GCC AGA GCC AGA CTG TGA TCC GTCGGATGTC
GLN GLY GLU SER ASN GLN GLU ARG GLY ALA ARG ALA ARG LEU ---

     550                                           600
CAAAAGGTCG CCACTTTTCC ACTTCCTGTT TCATTCTCGT TCCTTAAGAA AGTTACACAG GAATAGTATC

                              650
GAATCCTTGC CGAGTTTGAC TCAATTAGTT CAGTCAGTTT CAGGATATTA GTCATCTCTA CATTGATTAT

              700                                              750
GAGTATTCAG AAATTCCTTA AATATTCTGA CAAATGCTCT TTCCCTAAAC TCCCCCCATA AAAAAACCCG

     760
CCGAAGCGGG TTTTT

FIG. 3A

mRNA from Plasmid pTrp-BssH II-HuGRF

```
                                                50
AAGTTCACGT AAAAAGGGTA TCGACA ATG AAA GCA ATT TTC GTA CTG AAA GGT TCA CTG GAC
                          Met Lys Ala Ile Phe Val Leu Lys Gly Ser Leu Asp

                                     100
AGA GAT CTC GAC AGC CGT ATT GAA CTG GAA ATG CGT ACC GAT CAT AAA GAG CTG TCT GAA
Arg Asp Leu Asp Ser Arg Ile Glu Leu Glu Met Arg Thr Asp His Lys Glu Leu Ser Glu

                                150
CAT CTG ATG CTG GTT GAT CTC GCC CGT AAT GAT CTG GCA CGC ATT TGC ACC CCC GGC AGC
His Leu Met Leu Val Asp Leu Ala Arg Asn Aso Leu Ala Arg Ile Cys Thr Pro Gly Ser

             200
CGC TAC GTC GCC GAT CTC ACC AAA GTT GAC CGT TAT TCC TAT GTG ATG CAC CTC GTC TCT
Arg Tyr Val Ala Asp Leu Thr Lys Val Asp Arg Tyr Ser Tyr Val Met His Leu Val Ser

    250                                                                  300
CGC GTA GTC GGC GAA CTG CGT CAC GAT CTT GAC GCC CTG CAC GCT TAT CGC GCC TGT ATG
Arg Val Val Gly Glu Leu Arg His Asp Leu Asp Ala Leu His Ala Tyr Arg Ala Cys Met

                                                     350
AAT ATG GGG ACG TTA AGC GGT GCG CCG AAA GTA CGC GCT ATG CAG TTA ATT GCC GAG GCG
Asn Met Gly Thr Leu Ser Gly Ala Pro Lys Val Arg Ala Met Gln Leu Ile Ala Glu Ala

                                400
GAA GGT CGT CGC CGC GGC AGC TAC GGC GGC GCG GTA GGT TAT TTC ACC GCG CAT GGC GAT
Glu Gly Arg Arg Arg Gly Ser Tyr Gly Gly Ala Val Gly Tyr Phe Thr Ala His Gly Asp

                          450
CTC GAC ACC TGC ATT GTG ATC CGC TCG GCG CTG GTG GAA AAC GGT ATC GCC ACC GTG CAA
Leu Asp Thr Cys Ile Val Ile Arg Ser Ala Leu Val Glu Asn Gly Ile Ala Thr Val Gln

             500
GCG GGT GCT GGT GTA GTC CTT GAT TCT GTT CCG CAG TCG GAA GCC GAC GAA ACC CGT AAC
Ala Gly Ala Gly Val Val Leu Asp Ser Val Pro Gln Ser Glu Ala Asp Glu Thr Arg Asn

         550           .BssH II                                          600
AAA GCC CGC GCT GTA CTG CGC GCT ATG TAC GCT GAT GCT ATA TTC ACT AAC TCT TAC CGC
Lys Ala Arg Ala Val Leu Arg Ala Met TYR ALA ASP ALA ILE PHE THR ASN SER TYR ARG

                                                          650
AAG GTT CTG GGC CAA TTA TCA GCA CGC AAG CTT CTG CAG GAC ATT CTT AGT CGA CAA CAG
LYS VAL LEU GLY GLN LEU SER ALA ARG LYS LEU LEU GLN ASP ILE LEU SER ARG GLN GLN

                                     700
GGC GAA TCT AAC CAG GAG CGT GGC GCC AGA GCC AGA CTG TGA TCCGTC GGATGTCCAA
GLY GLU SER ASN GLN GLU ARG GLY ALA ARG ALA ARG LEU ---

                   750
AAGGTCGCCA CTTTTCCACT TCCTGTTTCA TTCTCGTTCC TTAAGAAAGT TACACAGGAA TAGTATCGAA

    800                                                     850
TCCTTGCCGA GTTTGACTCA ATTAGTTCAG TCAGTTTCAG GATATTAGTC ATCTCTACAT TGATTATGAG

                             900
TATTCAGAAA TTCCTTAAAT ATTCTGACAA ATGCTCTTTC CCTAAACTCC CCCCATAAAA AAACCCGCCG

    940
AAGCGGGTTT TT
```

FIG. 3B

Purification of HGRF_{Leu27} from E. coli Cells
Producing a TrpLE-HGRF Fusion Protein

**CELLS**

| Octonol, N₂ gas
| Sonicate

**CELL SONICATE**

| Centrifuge

**LOW SPEED PELLET** ———————————— CYTOSOL

| 70% Formic Acid
| Octonol, N₂ gas

**PRE-CNBr SOLUTION**

| CNBr, 6hr
| Rotory Evaporate
| Lyophilize
| Extract with 1N HAc
| Centrifuge

**HAc EXTRACT**

|

**PELLET**

| Dialize
| Centrifuge

**DIALYSATE** ———————————— PELLET

|

**CM-CELLULOSE POOL**

|

**HPLC POOL**

|

**G-50 POOL**

| Lyophilize 3-4 times

**FINAL PRODUCT — HuGRF_{Leu27}**

FIGURE 4

pTrp.LE-HuGRF

SstII BssHII Trp LE

FIGURE 5

FIGURE 6

FIGURE 7

FIGURE 8

A. **Stained Gel**

B. **Western Blot**

FIGURE 9

FIGURE 10

0212531

12/12

Sequence #:   1
Inject time:11:41:15
Date 1/ 8/86
File: An20-40-60S.N001

Operator: MM
Method: AN20-40-60S
Detector: 280
Column: ANAL
Flow: 1.5
Mobile Phase:ACN/H20/TfA

Run time: 37.00
Peak width: 0.25
Peak sensitivity: 0.05

FIGURE   11

European Patent Office

**PARTIAL EUROPEAN SEARCH REPORT**
which under Rule 45 of the European Patent Convention
shall be considered, for the purposes of subsequent
proceedings, as the European search report

0212531
Application number

## DOCUMENTS CONSIDERED TO BE RELEVANT

EP 86111097.1

| Category | Citation of document with indication, where appropriate, of relevant passages | Relevant to claim | CLASSIFICATION OF THE APPLICATION (Int. Cl.4) |
|---|---|---|---|
| X | EP - A1 - 0 133 282 (HOECHST AKTIENGESELLSCHAFT)<br>* Page 17; claim 1 *<br>-- | 1,14 | C 07 K 7/10<br><br>C 12 P 21/02<br><br>C 12 N 15/00<br><br>A 61 K 37/43 |
| A | EP - A1 - 0 108 387 (F.HOFFMANN - LA ROCHE & CO.)<br>* Fig. 2 *<br>-- | 1 | |
| A | EP - A2/A3 - 0 121 764 (F.HOFFMANN- LA ROCHE & CO.)<br>* Claims *<br>-- | 1,2,13, 14 | |
| D,A | EP - A1 - 0 146 210 (THE SALK INSTITUTE FOR BIOLOGICAL STUDIES)<br>* Claims 1,11 *<br>& US-A-4 518 586<br>-- | 1,13, 14 | |

**TECHNICAL FIELDS SEARCHED (Int. Cl.4)**

C 07 K

C 12 P

C 12 N

A 61 K

## INCOMPLETE SEARCH

The Search Division considers that the present European patent application does not comply with
the provisions of the European Patent Convention to such an extent that it is not possible to carry
out a meaningful search into the state of the art on the basis of some of the claims.
Claims searched completely:       1-9,13,14
Claims searched incompletely:     –
Claims not searched:              10-12
Reason for the limitation of the search:

(Article 52(4) EPC)

| Place of search | Date of completion of the search | Examiner |
|---|---|---|
| VIENNA | 14-11-1986 | WOLF |

European Patent
Office

| Category | Citation of document with indication, where appropriate, of relevant passages | Relevant to claim |
|---|---|---|
| **DOCUMENTS CONSIDERED TO BE RELEVANT** | | |
| D,A | EP - A2 - 0 138 416 (ELI LILLY AND COMPANY) <br> * Claims 1-3,13; pages 10,11 * <br> -- | 1,2 |
| P,A | EP - A1 - 0 176 341 (ELI LILLY AND COMPANY) <br> * Claims 1-3 * <br> ---- | 2-4,6-9 |

CLASSIFICATION OF THE APPLICATION (Int. Cl.4)

TECHNICAL FIELDS SEARCHED (Int. Cl.4)

EPO Form 1505.3   06.78